# EUROPEAN PATENT SPECIFICATION

(11) **EP 2 211 938 B1**
(45) Date of publication and mention of the grant of the patent: **28.01.2015**
(21) Application number: 08839207.1
(22) Date of filing: 17.10.2008
(51) Int. Cl.: A61M 5/00, A61M 39/10, A61M 39/26

(54) **DEVICES AND ASSEMBLIES FOR CONTROLLING FLUID FLOW**
VORRICHTUNGEN UND ANORDNUNGEN ZUR KONTROLLE DES FLÜSSIGKEITSDURCHFLUSSES
DISPOSITIFS ET ENSEMBLES DE RÉGULATION DE L'ÉCOULEMENT D'UN FLUIDE

(30) Priority: 19.10.2007 US 981233 P
(43) Date of publication of application: 04.08.2010
(73) Proprietor: Infusion Innovations, Inc., La Jolla, CA 92037 (US)
(72) Inventor: STROUP, David, K., El Cajon CA 92020 (US)
(74) Representative: Boult Wade Tennant
(86) International application number: PCT/US2008/080375
(87) International publication number: WO 2009/052433

(56) References cited:
- WO-A1-01/93936
- US-A- 5 954 313
- US-A- 6 050 978
- US-A1- 2003 199 835
- US-B1- 6 364 869
- US-B2- 7 118 560

## Description

### FIELD OF THE INVENTION

The present invention relates generally to control of flow. More particularly, the present invention relates to devices, assemblies, and/or methods for controlling fluid flow, e.g., to connectors and/or valves for controlling flow through an IV or other fluid line into a patient.

### BACKGROUND

Controlling flow is an important and useful tool in virtually all scientific fields. One such field where controlling flow is highly useful is in the medical arena. For example, it may be useful to control flow during infusion, e.g., when introducing fluid into a blood vessel, such as a vein, via a fluid line for therapeutic and/or diagnostic purposes. The fluid introduced may be saline solution, plasma solution, glucose solution, antibiotics, pain relievers, nuclear medicine agents, and the like. Infusion may involve many fluid doses into a patient over long periods of time. Early in the infusion field, each fluid dose required a new needle to be inserted into the vein. Repeated insertion of a needle into the same vein of a patient, however, may damage the vein, increase the potential for bruising, and/or inflict pain on or discomfort to the patient.

Health professionals quickly changed this routine by inserting one needle into the patient's vein, and leaving it there for initial and subsequent fluid dose introductions. This stationary needle could be connected to a first or proximal end of a catheter that had an opening at a second or distal end for receiving fluid from a syringe or other device. For example, a latex cap was placed over the distal end of the catheter, which could be penetrated by a beveled hollow needle. Once inserted into the patient's vein, the stationary needle could be secured with tape, but was prone to disconnection from the patient. From this basic concept a range of needleless connectors were developed capable of linking the fluid line to the patient's catheter directly thereby bypassing needle use. Further industry directive and federal regulation encouraged this alternative technique of promoting needleless connectors use thereby promoting removal of sharp instruments from the patient area.

Early needleless connectors featured a split septum on the female end (e.g., the end closer to the patient during connection). The split septum could be opened by inserting a cannula. The male end featured a blunt cannula, which was inserted into the split-septum on the female end. This method relieved some of the disconnection problems, but a new problem emerged. Removing the blunt cannula created a negative pressure inside the catheter, which caused a small amount of blood from the patient to flow into the proximal end of the catheter. These small amounts of blood would accumulate in the catheter, thereby clogging the fluid pathway. The consequence of this negative pressure, or negative bolus effect, was to require a new, clean catheter. The replacement of these clogged catheters may be expensive and/or painful to the patient.

The split septum on the female end was then replaced with an anti-reflux valve activated by the use of a male-female Luer configuration, also termed sequential valving. This male-female Luer connection has been standardized by the industry, e.g., through international standard ISO 594-2 "Conical fittings with a 6 % (Luer) taper for syringes, needles and certain other medical equipment", Part 2: Lock fittings. The anti-reflux design helped stabilize the pressure difference during disconnection, and connectors exist today that allow a user to adjust the amount of pressure upon disconnection to attempt to create one of the following: a positive, negative, or zero bolus effect.

Hence, needleless medical connectors for fluid injection into or out of an intravenous (IV) system are now well understood and widely used in the healthcare practice. The demand for closed needleless systems for fluid administration is driven by the safety concerns associated with medications that are toxic to healthcare workers that prepare and administer these medications. These medications include chemotherapy and radiotherapeutic agents. Key industry organizations such as the National Institute for Occupational Safety and Health (NIOSH), Oncology Nursing Society (ONS) and American Society of Health System Pharmacists (ASHP) recommend adopting closed systems to minimize drips, leaks, or spills of the drug to help eliminate surface contamination and exposure.

The vast majority of the self-sealing medical connectors that are used for the administration of parenteral fluids are designed with an unsealed male Luer connector on the end that remains connected to the patient's IV line, fluid source, etc., and a female connector on the opposite free end of the connector through which a syringe or other types of devices is connected. In many devices on the market, there is a self sealing valve built into the female connector. The male Luer typically does not have an internal valve, and as such, any remaining fluid is capable of being exposed to the care providers and patients upon disconnection of the unsealed male Luer. As mentioned above, for certain applications, the fact that residual volume of the fluid may be unsealed and/or exposed to individuals around the IV system may pose significant health hazards. Additionally, these conventional Luer connectors may have a larger internal volume in which fluid may collect, and also employ many parts thereby increasing the potential for error in manufacturing or during use.

The standard connection mechanism for these Luer connectors involves aligning the threads together by a helical threading action. This threading action is meant to establish a connection between (e.g., engage) the two Luer ends, and is not the force used to open or close (e.g., actuate) fluid pathways. As the two Luer connectors are being connected together, there is a separate translational (e.g., on a vertical axis) action within these connection assemblies that acts to engage the fluid pathways. Traditionally, the female end has a thread on the outside while the male has a thread on the inside. Since most female ends have self sealing valves, the user may open the fluid path with the translational force during engagement or after the male end is completely engaged and locked inside the female end. Thus, the user may not know at what point the fluid path is sufficiently opened or closed during connection and disconnection of the two connectors. The user only knows that the fluid path is closed (e.g., the two connectors are deactuated), when the two connectors are completely disengaged, or disconnected, and separated.

Thus, there is a need in the art for a connector and/or connecting assembly that may effectively avoid uncertainty in the actuation process, avoid certain undesired pressure effects, create certain desired pressure effects, reduce the internal volume of the assemblies, and/or decrease the number of members required for manufacturing.

WO 01/93936 relates to a needle-less medical connector for blood transfer, intravenous fluid supply, medication dosage and the like. The connector has a longitudinal axis along which relatively rotatable parts are aligned and interconnected. The rotation of these parts opens and closes a flow path through the connector.

### SUMMARY OF THE INVENTION

The present invention provides a connector assembly according to claim 1.

The present invention is directed to apparatus and methods for controlling flow. through a fluid line, for example, to connectors and/or valves for delivering fluid via an intravenous ("IV") or other medical fluid line into a patient.

Conventional devices and assemblies for establishing medical connections are not completely effective and are potentially unsafe. For example, conventional medical connectors may expose the user to harmful agents during disconnection as a result of undesired bolus effects, may collect undesired fluid within their internal volumes after disconnection, may not notify the user of the actuation status during connection and disconnection, and/or may include many parts thereby making manufacture expensive. In contrast, embodiments herein may use fewer parts and hence may minimize and/or eliminate residual fluid within the connectors after disconnection, may utilize a rotational actuation force as opposed to translation force to avoid or create a desired bolus effect, and/or may incorporate actuation status indicators to notify the user when actuation is complete.

In exemplary embodiments, medical connectors disclosed herein may be used for the administration of parenteral fluids, such as needleless connectors that may offer alternative mechanisms to conventional Luer connectors, may utilize a visual indicator that provides instant feedback to an operator regarding actuation status, and/or may employ alternative ways for energy storage, including rotational force, electromagnetic, polymer torsion spring, and/or spring washers for actuation.

As used herein, "proximal" refers to a first end of the device, e.g., the portion of the device or component that is closer to the patient when the device or component is properly positioned, for example, on a patient's IV line. "Distal" refers to a second opposite end of the device, e.g., the portion of the device or component that is farther from the patient when the device or component is properly positioned, for example, on a patient's IV line. For reference, the female end may be upstream in an IV flow circuit and the male may be downstream or vice versa. "Actuated" refers to the condition in which the fluid path is opened to allow fluid to transfer freely along the fluid path, while "deactuated" refers to the condition in which the fluid path is closed and fluid transfer is not permitted. "Engaged" refers to the condition in which two members that are designed for connection, for example, Luer connectors, are physically connected to each other in a manner in which they are designed to be connected, while "disengaged" refers to the condition in which two members, for example, Luer connectors, are physically disconnected from one another. When two members are referred to as "engaged," they may or may not be "actuated." The two members are "actuated" only when they are fully engaged, and fluid transfer is permitted between them. Alternatively, one member may use one valve component (male or female) and a passive (non-valved) element of opposite gender. "Female" Luer connector refers to a connecting member that includes a Luer thread on its outer surface. "Male" Luer connector refers to a connecting member that includes a Luer thread on its inner surface. "Passive" refers to the conditions under which a connector or assembly functions, and signifies that the assembly is capable of deactuating automatically as it is disengaged. "Non-Passive" refers to the conditions under which a connector or assembly functions, and signifies that the assembly does not automatically deactuate as it is disengaged, but requires a separate action. Optionally, in the embodiments herein, there may be sequential valving, resulting in co-dependent or independent actuation of male and/or female sides of valves.

In accordance with an exemplary embodiment, a connector assembly is provided for controlling flow in a fluid line. The connector assembly may include an outer shell, an inner housing, and a tubular member. The outer shell may include a first end, a second end, and a passage extending therebetween. The inner housing is disposed within the outer shell and may include a boss disposed adjacent the first end. The inner housing may be movable axially within the outer shell between a first position adjacent the first end and a second position further from the first end than the first position when a device is connected to the first end of the outer shell.

The tubular member may be disposed within the inner housing and may include a fluid passage therein extending between the second end of the outer shell and the boss of the inner housing. The tubular member may be carried by the inner housing such that the tubular member moves axially as the inner housing moves between the first and second positions. The connector assembly may also include cam features on the outer shell and/or the tubular member for causing the tubular member to rotate as the inner housing moves between the first and second positions, thereby opening a fluid path between the fluid passage in the tubular member and the first end of the outer shell.

For example, when a device is connected to the first end of the outer shell, the tubular member may rotate relative to the inner housing to allow fluid flow through the assembly with the device connected to the first end of the outer shell. Optionally, the inner housing may be biased to the first position, e.g., to bias the tubular member to close the fluid path.

In accordance with another embodiment, a connector and/or valve assembly is provided for controlling flow in a fluid line that includes an outer shell including a first end, a second end, and a passage extending therebetween, the first end including a connector for connecting the assembly to a fluid line; an inner housing disposed within the outer shell and including a boss disposed adjacent the first end, the inner housing being movable axially within the outer shell between a first position adjacent the first end and a second position further from the first end than the first position when a device is connected to the connector on the first end of the outer shell; a tubular member disposed within the inner housing and including a fluid passage therein extending between the second end of the outer shell and the boss of the inner housing, the tubular member carried by the inner housing such that the tubular member moves axially as the inner housing moves between the first and second positions; and cam features on the inner housing and the tubular member for causing the tubular member to rotate as the inner housing moves between the first and second positions, thereby opening a fluid path between the fluid passage in the tubular member and the first end of the outer shell to allow fluid flow through the assembly with the device connected to the first end of the outer shell.

Methods for using such connector and/or valve assemblies are also provided.

Other aspects and features of the present invention will become apparent from consideration of the following description taken in conjunction with the accompanying drawings.

### BRIEF DESCRIPTION OF THE DRAWINGS

The drawings illustrate exemplary embodiments of the invention, in which:
Figure 1A is a perspective view of an exemplary embodiment of a passive connecting valve assembly including an outer shell or bezel with a Luer thread and status windows, and a section of tubing extending from a distal end of the outer shell.
Figure 1B is an exploded view of the passive connecting assembly of Figure 1A, showing the outer shell with a proximal throughbore including a Luer thread, a distal fluid path and status windows; a covering body including a male boss with a deformable membrane and an outer mating surface including status indicators; a shaft with an elastic member and a mating member; a backing member with cam elements for interacting with the mating member, and the section of tubing.
Figure 2 is a perspective view of an exemplary embodiment of the outer shell of the connecting assembly of Figures 1A and 1B that includes proximal and distal throughbores, a proximal Luer thread, and status windows.
Figure 3 is a perspective view of an exemplary embodiment of the covering body of the connecting assembly of Figure 1B that includes a male boss with a deformable membrane, an outer mating surface possessing status indicators, recesses on a distal side of the outer mating surface for receiving an elastic member, and a throughbore.
Figure 4A is a perspective view of an exemplary embodiment of the shaft of the connecting assembly of Figure 1B that includes a conduit, a fluid post with openings, and a mating member with pegs on its proximal side and cam features on its distal side.
Figure 4B is a cross-section of the shaft of Figure 4A taken along line 4B-4B.
Figure 5 is a perspective view of an exemplary embodiment of the backing member of the connecting assembly of Figure 1B that includes cam elements, a throughbore, and proximal and distal end segments.
Figures 6A(1) and 6A(2) are perspective views of the connecting assembly of Figures 1A and 1B in a deactuated condition, with the outer shell included and removed, respectively, showing a Luer connector mating with the outer shell; the covering body with status indicators that indicate the deactuated condition; the cam features of the shaft; and the backing member with its cam elements spaced apart from the cam features.
Figure 6B is a cross-sectional view of the deactuated connecting assembly shown in Figures 6A(1) and 6A(2), taken along line 6B-6B.
Figure 6C is a cross-sectional view of the deactuated assembly shown in Figures 6A(1) and 6A(2), taken along line 6C-6C.
Figures 6D(1) and 6D(2) are perspective views of the connecting assembly of Figures 1A and 1B in an actuated condition, with the outer shell included and removed, respectively, showing the Luer connector; the covering body status indicators that indicate the actuated condition; the cam elements on the shaft engaging with the cam elements on the backing member.
Figure 6E is a cross-sectional view of the actuated connecting assembly shown in Figures 6D(1) and 6D(2), taken along line 6E-6E.
Figure 6F is a cross-sectional view of the actuated assembly shown in Figures 6D(1) and 6D(2), taken along line 6F-6F.
Figure 6G is a perspective view of the deactuated connecting assembly shown in Figures 6A(1) and 6A(2) with the outer shell shown in phantom for clarity.
Figure 6H is a perspective view of the actuated connecting assembly shown in Figures 6D(1) and 6D(2) with the outer shell shown in phantom for clarity.
Figure 7A is a perspective view of another embodiment of a passive connecting assembly including an outer shell with a male Luer thread connected to an distal end of a length of tubing.
Figures 7B and 7C are exploded views of the assembly of Figure 7A, including an outer shell with a proximal throughbore containing a Luer thread and a distal throughbore; a covering body including a male boss with a deformable membrane and an outer mating surface; a shaft with an elastic member and a mating member; and a backing member.
Figure 8 is a perspective view of the outer shell of the assembly of Figures 7A-7C.
Figures 9A and 9B are perspective views of the shaft of the assembly of Figures 7A-7C.
Figures 10A and 10B are perspective views of components of a backing member of the assembly of Figures 7A-7C.
Figure 11A is a side view of the assembly of Figures 7A-7C in a deactuated condition before a Luer fitting has been connected to the assembly.
Figures 11B and 11C are cross-sectional views of the assembly of Figure 11A, taken along lines 11B-11B and 11C-11C, respectively.
Figure 11D is a side view of the assembly of Figure 11A with the outer shell shown in phantom.
Figure 12A is a side view of the assembly of Figures 7A-7C in an actuated condition after the Luer fitting has been connected to the assembly.
Figures 12B and 12C are cross-sectional views of the assembly of Figure 12A, taken along lines 12B-12B and 12C-12C, respectively.
Figure 12D is a side view of the assembly of Figure 12A with the outer shell shown in phantom.
Figures 13A-13C are perspective views of yet another embodiment of a valve/connector assembly including a female Luer fitting, shown before and after connection to a male Luer fitting.
Figures 14A and 14B are exploded perspective views of the assembly of Figures 13A-13C.
Figures 15A and 15B are cross-sectional views of the assembly of Figures 13A-13C before and after actuation, respectively.
Figures 16A and 16B are cross-sectional views of the assembly of Figures 15A and 15B, taken along lines 16A-16A and 16B-16B, respectively.
Figure 17A is a perspective view of still another embodiment of a passive connecting assembly including an outer shell with a male Luer thread connected to an distal end of a length of tubing.
Figures 17B and 17C are exploded views of the assembly of Figure 17A, including an outer shell with a proximal throughbore containing a Luer thread and a distal throughbore; a covering body including a male boss with openings and an outer mating surface; a shaft with an elastic member and a mating member; a camming element; and a backing member.
Figure 18 is a perspective view of the outer shell of the assembly of Figures 17A-17C.
Figure 19 is a perspective view of the covering body of the assembly of Figures 17A-17C.
Figure 20 is a perspective view of the shaft of the assembly of Figures 17A-17C.
Figure 21A is a perspective view of a backing member of the assembly of Figures 17A-17C.
Figure 21B is a perspective view of a camming element of the assembly of Figures 17A-17C.
Figure 22A is a side view of the assembly of Figures 17A-17C in a deactuated condition before a Luer fitting has been connected to the assembly.
Figures 22B and 22C are cross-sectional views of the assembly of Figure 22A, taken along lines 22B-22B and 22C-22C, respectively.
Figure 22D is a side view of the assembly of Figure 22A with the outer shell removed to show the position of the internal components of the assembly before actuation.
Figure 23A is a side view of the assembly of Figures 17A-17C in an actuated condition after the Luer fitting has been connected to the assembly.
Figures 23B and 23C are cross-sectional views of the assembly of Figure 23A, taken along lines 23B-23B and 23C-23C, respectively.
Figure 23D is a side view of the assembly of Figure 23A with the outer shell removed to show the position of the internal components of the assembly after actuation.

### DETAILED DESCRIPTION OF THE EXEMPLARY EMBODIMENTS

Apparatus and methods described herein may relate to connecting devices and assemblies that, among other things: utilize a rotational force for actuation to avoid or create a desired bolus effect, minimize the number of parts necessary for manufacture, decrease the internal volume of the devices and assemblies, and/or use status indicators to signify when actuation and deactuation are complete. Also, in some embodiments, the apparatus described herein may create and maintain a flat (planar) surface that is easily swabbed for cleaning and sterilization purposes. Although embodiments of connecting devices and assemblies are described herein with respect to medical connections, such connecting devices and assemblies are not limited to medical connections alone but may be applicable to any connection device or assembly that could benefit from the use of a rotational actuation force, status indicators, and/or any of the other features described herein.

For the following description, it should be noted that correspondingly labeled structures across the figures (e.g., 132 and 232, etc.) may possess the same general characteristics and/or may be subject to the same basic structure and function.

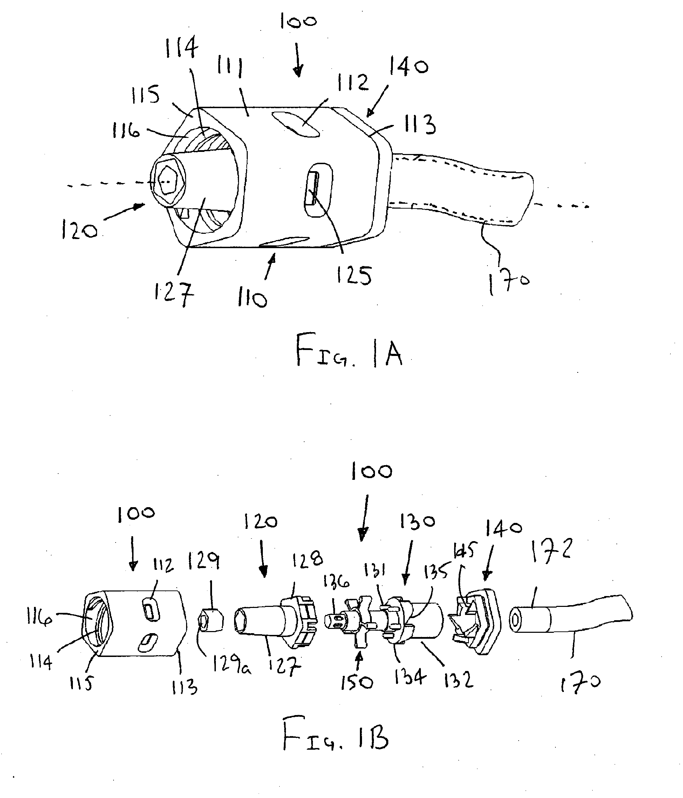

An exemplary embodiment of a passive connection assembly 100 is shown in Figures 1A and 1B that generally includes a shaft, core pin, conduit, or other tubular member 130, a backing member or cover 140, a covering body or inner housing 120, and an outer shell or bezel 110. As explained further below, the shaft 130 may interact with the covering body 120 and backing member 140 to provide a selectively opened fluid path through the assembly 100. The assembly 100 includes interacting cam elements and/or features, which may translate axial action, e.g., when the assembly 100 is connected to a fluid line, into rotational movement, or rotational action into axial movement to open and/or close the fluid path. The assembly 100 may be biased to close or open the fluid path, and the bias may be overcome by the axial or rotational action to open or close the fluid path. The outer shell 110 may include one or more windows 112 that may indicate when the assembly 100 is in open and/or closed conditions, and/or the assembly 100 may include other indicators that may indicate when the assembly 100 is in the open and/or closed conditions. The assembly 100 may include one or more Luer or other connectors, e.g., a male Luer thread 114 as shown on the outer shell 110, for connecting the assembly 100 to a fluid line (not shown). It will be appreciated that other connectors may also be provided instead of the Luer thread 114.

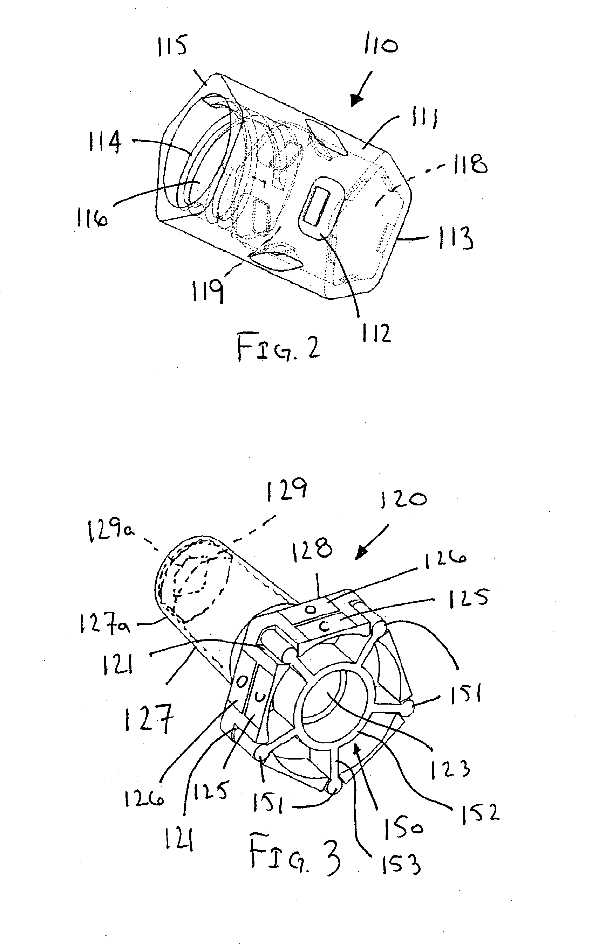

Figure 1B is an exploded view of the assembly 100 showing the outer shell 110, along with the covering body 120 with a deformable sleeve or membrane 129, the shaft 130 with an elastic member 150, and the backing member 140. The various components of the assembly 100 shown in Figure 1B are shown in further detail in Figures 2-5 and described further below.

Figure 2 shows the outer shell 110, which includes one or more status windows 112 in an outer surface 111 thereof, an unthreaded portion 115 with a distal throughbore 118, and a proximal throughbore 116, e.g., for receiving the covering body 120 and shaft 130 therethrough. The proximal throughbore 116 may include the male Luer thread 114 therein. As shown, the outer shell 110 includes a pentagon shaped outer surface 111, although alternatively the outer surface 111 may include other shapes, e.g., to facilitate a user easily gripping and/or manipulating the outer shell 110, for example, to engage the male Luer thread 114 with a female Luer connector (not shown) via a threading action. Exemplary shapes include a triangle, square, hexagon, heptagon, and other suitable polygons.

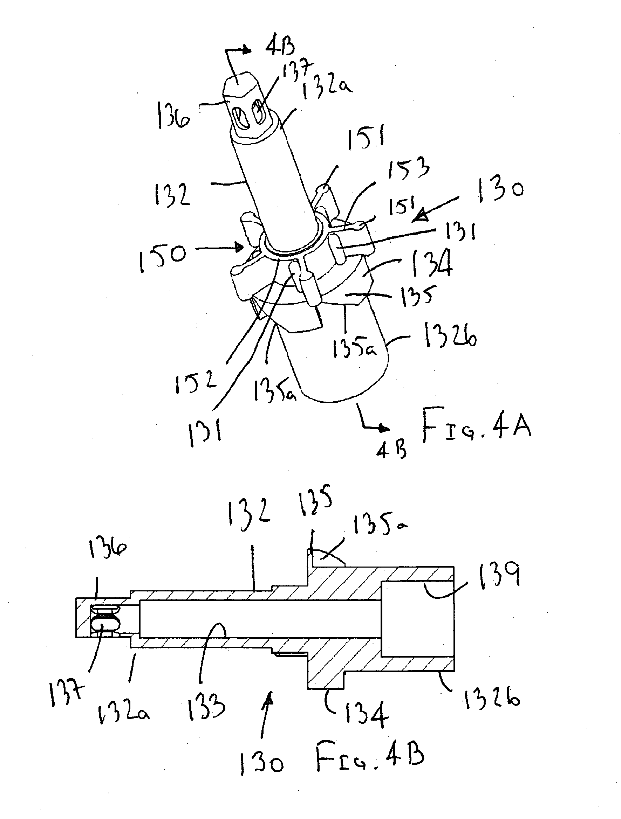

The distal throughbore 118 extends through the unthreaded portion 115 from distal end 113 of the outer shell 110 to location 119, which may be situated as shown at or near the midpoint of the length of outer shell 110, e.g., such that the distal throughbore 118 communicates with proximal throughbore 116. The longitudinal distance of distal throughbore 118 from the distal end 113 to location 119 is sufficient to accommodate the outer mating surface 128 of covering body 120 (See Figure 3) and shaft 130 (See Figure 4A).

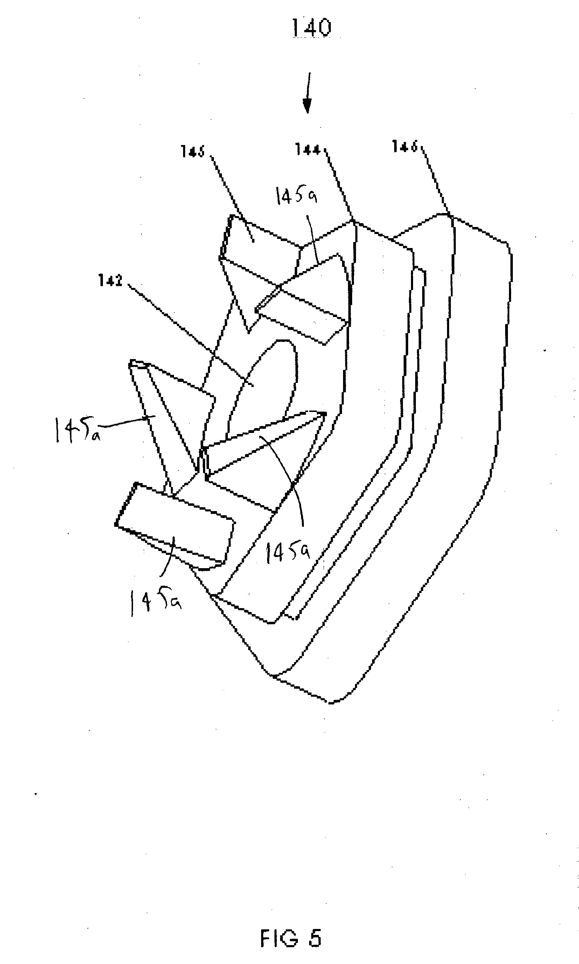

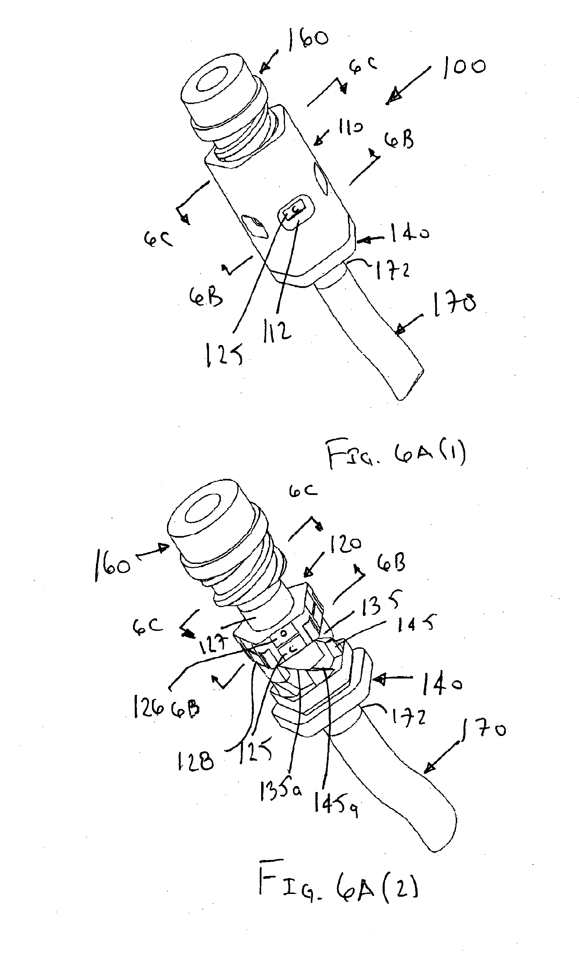

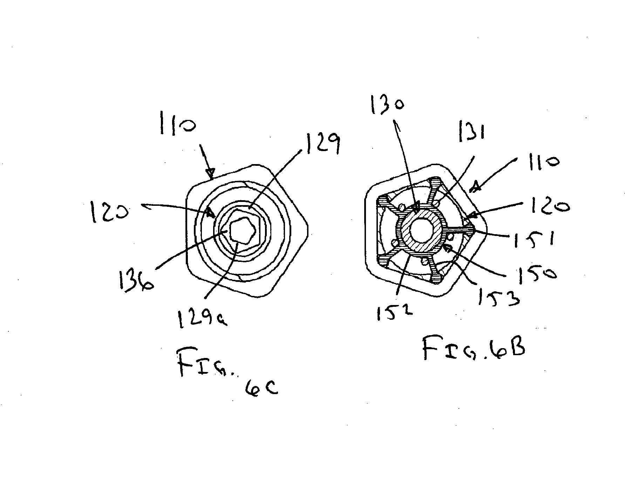

The distal throughbore 118 may be pentagonal in shape as shown, or it may be any other shape (e.g., a triangular, square, pentagonal, hexagonal, heptagonal, octagonal, or other polygonal shape) as long as it is able to receive, effectively contact, and limit movement of the covering body 120 (See Figure 3) and the backing member 140 (See Figure 5) relative to the outer shell 110 when the assembly 100 is being engaging, e.g., with a female Luer connector 160 of a fluid line (not shown, see Figures 6A(1) and 6A(2)). For example, this polygonal shape may ensure that the outer shell 110, covering body 120, and backing member 140 all rotate substantially in unison. The polygonal shape may also ensure that the backing member 140 does not move axially relative to the outer shell 110 and/or may allow limited axial motion of the covering body 120 within the outer shell 110. The same shape may be used for the distal throughbore 118 and the outer surface 111 of the outer shell 110, e.g., for manufacturing convenience, but the device may still function even if these shapes are different. The shape and size of the distal throughbore 118, however, should correspond substantially to the shape and size of both the outer mating surface 128 of the covering body 120 and proximal end segment 144 of the backing member 140, as explained further elsewhere herein. Thus, the outer shell 110, covering body 120, and backing member 140 rotate together, while the shaft 130 (See Figure 4A) may rotate independently of the other components, also as described further elsewhere herein.

The proximal throughbore 116 may be circular in shape and include the Luer threads 114, as shown in Figure 2. This facilitates mating the outer shell 110 with a female Luer connector 160 (not shown, see Figures 6A(1) and 6A(2)), e.g., when engaging the two connectors to couple the assembly 100 with an IV or other fluid line (also not shown). The longitudinal distance or depth of the proximal throughbore 116 is sufficient to accommodate the male boss 127 of the covering body 120 (See Figure 3) being received therethrough with the proximal end of the male boss 127 projecting past the proximal end 115 of the outer shell 110 when the male boss 127 is fitted within the proximal throughbore 116. The Luer thread 114 may have conventional dimensions, such as those of the ISO industry standards, e.g., to allow compatibility with standard Luer connectors. The diameter of the distal throughbore 118 may be large enough to accommodate receiving the covering body 120 therethrough, to make contact with and mate with the outer mating surface 128 (See Figure 3) of the covering body 120, and/or to fit over and mate with the proximal end segment 144 of the backing member 140.

The status windows 112 may be arranged circumferentially around outer surface 111 of the outer shell 110, as shown in Figure 2. The windows 112 may be rectangular in shape as shown, or may have any other shape including but not limited to circles, triangles, etc., as long as the windows 112 may fit over and reveal deactuated status indicators 125 or actuated status indicators 126 on the surface of the covering body 120 (See Figure 3). Although five windows are shown in Figure 2, any number of windows may be provided, e.g., that allow a user to easily see at least one status indicator 125, 126 during either actuation or deactuation. The status windows 112 may be located on the outer surface 111 at or near a mid-point along a length of the outer shell 110, or they may be located elsewhere on the outer surface 111 of the outer shell 110 as long as they are capable of revealing one or more of the status indicators 125, 126 when in the actuated and/or deactuated states. It will be appreciated that the assembly 100 may include other status indicators (not shown) in addition or instead of the status windows 112 and status indicators 125, 126, as described elsewhere herein.

Figure 3 shows the covering body 120, which includes a male boss 127 with a deformable housing 129, a throughbore 123, and status indicators 125, 126 disposed on an outer mating surface 128. Also shown in Figure 3, the covering body 120 includes recesses 121 for receiving end hubs 151 of the elastic member 150, as described further below. Alternatively, the covering body 120 may include projections (not shown) around which an elastic band or member (not shown) may be secured. In an exemplary embodiment, the male boss 127 may be dimensioned per ISO industry standards, and may be cylindrical in shape with a tapered proximal end 127a. During assembly, the outer mating surface 128 may contact the distal throughbore 118 of the outer shell 110 at or near location 119 (not shown, see, e.g., Figure 2) when the covering body 120 is inserted into the outer shell 110 (See Figures 1A and 1B). The outer mating surface 128 may have the same shape and size as the distal throughbore 118 of outer shell 110 in order for the outer mating surface 128 to fit within and make contact with the distal throughbore 118 of the outer shell 110, i.e., to allow axial movement but prevent rotation of the covering body 120 within the outer shell 110.

The status indicator(s) 125, 126 may be seen in Figure 3 on the outer mating surface 128. The status indicator(s) 125, 126 may signify to a user when actuation and/or deactuation is complete, as described elsewhere herein. The color or design of the deactuated status indicator(s) 125 may represent deactuation or a "closed" no-flow condition for the assembly 100 while the color or design of the actuated status indicator(s) 126 may represent actuation or an "open" or flow condition for the assembly 100. For simplicity, the deactuated status indicator 125 is shown with the letter "C" (for "closed") while the actuated status indicator 126 is shown with the letter "O" (for "open"), although color indicators may be more easily identified during use (e.g., red for closed and green for open). These indicators 125, 126 may be painted, glued, etched, and/or attached to the outer mating surface 128 via any method known in the art. The status indicators 125, 126 may have very minimal thickness so as not to increase the girth of the outer mating surface 128, i.e., to avoid any increased resistance between the outer mating surface 128 and the outer shell 110.

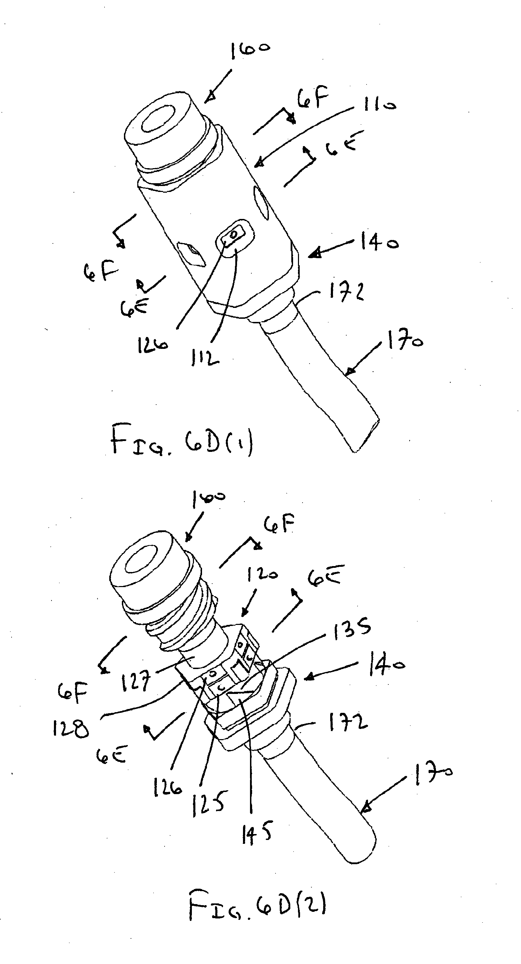

As the fluid path is actuated during use, the status windows 112 on the outer shell 110 may reveal one or more actuated status indicators 126 (see, e.g., Figure 6D(1)). This signifies that actuation is complete and that the user should not detach the two mated Luer connectors 114, 160 (unless it is desired to close the fluid path). When the user wishes to close the fluid path, the user may unthread the two Luer connectors and when the status windows 112 reveal one or more deactuated status indicators 125, then deactuation is complete (see, e.g., Figure 6A(1)). The actuation and deactuation processes are described further below with respect to the other structural components. It will be appreciated that, optionally, only one set of status indicators may be provided, if desired (not shown), e.g., to indicate only the actuated or deactuated state, rather than the two sets shown.

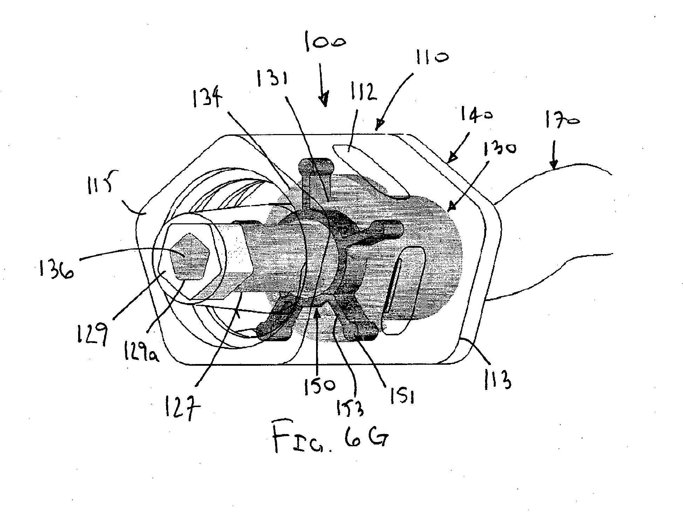

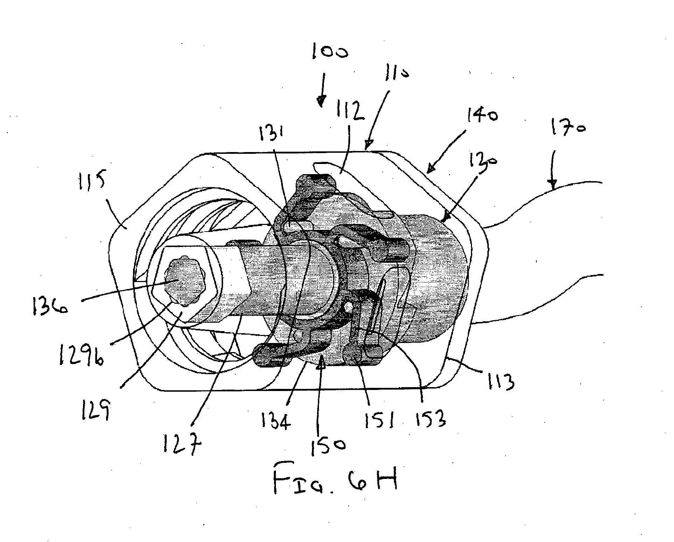

The recesses 121 may be located at each corner of the distal side of the outer mating surface 128 (e.g., forming apices of the star-shape), as shown in Figure 3. The recesses 121, along with their counterpart pegs 131 described further below, hold elastic member 150 in place. The recesses 121 may have a height such that they are capable of stably holding the hubs 151 of the elastic member 150 in place. As shown, the elastic member 150 includes a central annular base 152 from which spokes 153 extend to the hubs 151. The hubs 151 may be securely received in the recesses 121, e.g., by using an interference fit, bonding with adhesive, sonic welding, and the like. The spokes 153 may be used to store energy, i.e., to bias the elastic member 150, and consequently the shaft 130 towards a position corresponding to the closed (or optionally open) condition. For example, as explained elsewhere herein (and shown in Figures 6G and 6H), during use, e.g., when connecting and/or opening the fluid path of the assembly 100, the pegs 131 may deform the spokes 153 radially when the shaft 130 is rotated, thereby storing energy, which may be released when the assembly 100 is closed and/or disconnected.

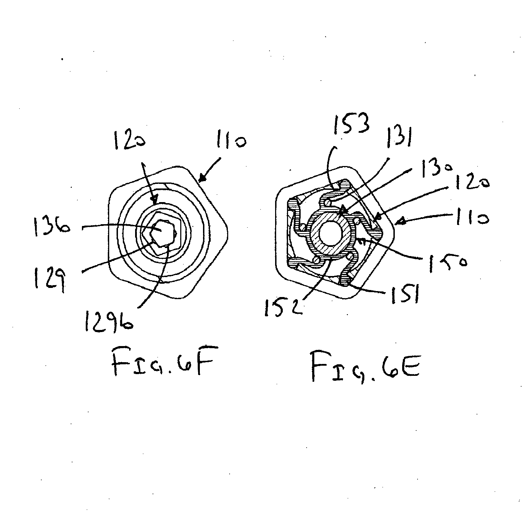

The elastic member 150 may be composed of any commonly used material in the art such that it is capable of maintaining a stable, and slightly stretched state (e.g., while in the undeformed star-shape shown in Figure 3), and thereafter being deformed to the configuration illustrated in Figure 6F. Thus, the elastic member 150 should be strong enough to avoid tearing or breakage when in the deformed state shown in Figure 6F, be elastic enough to pull itself back into the undeformed star-shape shown in Figure 3 when allowed, yet not be too resistant to prevent a user from manipulating it successfully between the two states (e.g., expanded and star-shapes). It will be appreciated that other energy storage devices or biasing mechanisms may be provided instead of the elastic member 150, e.g., an elastic band, spring, and the like, as described elsewhere herein.

The throughbore 123 runs the entire length of the covering body 120, e.g., from the outer mating surface 128 through the male boss 127 to the deformable membrane 129, as shown in Figure 3. The throughbore 123 may be circular and/or have a diameter exactly or substantially equal to the outer diameter of the conduit 132 of the shaft 130 (not shown, see, e.g., Figure 4A). The deformable membrane 129 may be located inside the proximal end of the throughbore 123, as shown in Figure 3, or may be disposed over the proximal end of the throughbore 123 (not shown). The deformable membrane 129 may have a polygonal shaped hollow center 129a that matches the size and shape of fluid cap 136 of the shaft 130.

The deformable membrane 129 is fixedly attached to the male boss 127, e.g., about its outer circumference, such that the opening 129a in the deformable membrane 129 remains substantially stationary while the fluid cap 136 of the shaft 130 rotates, i.e., is capable of being deformed while remaining attached to the male boss 127, as described elsewhere herein. For example, the proximal end 127a of the male boss 127 may have a polygonal or other noncircular recess therein and the outer surface of the deformable membrane 129 may have a similar shape. This may provide an interference fit when the deformable membrane 129 is inserted into the male boss 127, i.e., preventing rotation of the deformable membrane 129 relative to the male boss 127. Optionally, the deformable membrane 129 may be attached to the male boss 127 by bonding with adhesive, sonic welding, fusing, and the like in addition to or instead of the interference fit. The deformable membrane 129 is durable enough to resist ripping or tearing during actuation, yet strong and resilient enough to assume its original polygonal shape after deactuation. Similar to the elastic member 150, the deformable membrane 129 may be composed of an elastomeric material, e.g., silicone, or any other suitable material capable of performing the functions described herein.

Figures 4A and 4B show the shaft 130, which includes a conduit or tubular body 132 including first and second ends 132a, 132b, a throughbore 133 (shown in Figure 4B) extending therebetween, a mating member 134 with cam features 135, pegs 131, and a fluid post 136 with one or more side openings 137 on the first end 132a. Optionally, the second end 132b may include one or more connectors (not shown), a length of tubing, and the like. As shown in Figure 4B, the second end 132b includes an enlarged recess 139, which may be sized for receiving an end 172 of a section of tubing 170 (not shown, see Figure 1B). The tubing 170 may be substantially permanently or removably received in the recess 139, e.g., using an interference fit, one or more mating connectors (not shown), bonding with adhesive, sonic welding, and the like. The shaft 130 and the mating member 134, together with their respective components may be substantially permanently connected to one another, e.g., by integrally molding the shaft 130, the mating member 134, and pegs 133 from a single piece, by forming the components separately and attaching them together, e.g., by interference fit, mating connectors, bonding with adhesive, sonic welding, and the like.

Also shown in Figure 4A is the elastic member 150, which is the same as elastic member 150 shown in Figure 3, but here it is shown with the pegs 131 disposed adjacent to and/or between the spokes 153. Thus, the elastic member 150 resides both around the shaft 130, e.g., above the proximal surface of the mating member 134, and on the distal underside of the outer mating surface 128 (not shown, see Figure 3), e.g., when the shaft 130 is inserted into the covering body 120 (e.g., before or after inserting the covering body 120 into the outer shell 110), e.g., for elastically coupling the covering body 120 with the shaft 130. Figure 4B also shows the throughbore 133 extending through the length of the conduit 132, which extends almost the entire length of the shaft 130. The fluid post 136 resides at the proximal end of the conduit 132 and the opening(s) 137 communicate with the throughbore 133. The fluid post 136 has a corresponding polygonal shape that matches the inside opening 129a of the deformable membrane 129 (not shown, see, e.g., Figure 6C). The polygonal shape used for the fluid post 136 is shown as a pentagon, but alternatively, any other geometric shape as described above for the outer mating surface 128 may be used for both the fluid post 136 and the opening 129a in the deformable membrane 129.

The outer mating member 134 is shown located near the midpoint of the conduit 132. The mating member 134 may be positioned along the external surface of the conduit 132 so that when the shaft 130 is inserted into the covering body 120, as shown in Figures 1A and 1B, the distal side of the outer mating surface 128 makes contact with the proximal side of the mating member 134. In this position, the recesses 121 and pegs 131 hold the elastic member 150 in the star-shape, as shown in Figures 3 and 4A. The star-shape assumes five recesses 121 on the covering body 120 and five pegs on the mating member 134, as well as five spokes 153 and hubs 151 on the elastic member 150 due to the pentagonal shape shown, but the number of recesses and pegs, and thus the shape of the elastic member 150 may be changed, e.g., to correspond with the selected polygonal shape used for the outer mating surface 128 and its matching components.

The pegs 131 are attached to and/or otherwise extend from the proximal side of the mating member 134, as shown in Figure 4A. The pegs 131 may couple rotational motion of the annular base 152 of the elastic member 150 to rotation of the shaft 130.

Triangular indentations or cam features 135 are located on the distal side of the mating member 134. These cam features 135 may mate with triangular projections, splines or other mating cam elements 145 located on the backing member 140 shown in Figure 5 in order to effect actuation. The angle of each hypotenuse of these cam components (e.g., cam features 135 and cam elements 145) may be changed to correspond to the selected polygonal shape of the fluid post 136 in order to maximize fluid flow upon actuation of the assembly 100. Also, the size and depth of the cam features 135 may be changed with the polygonal shape of the fluid cap 136 to successfully effect or maximize actuation flow. The relationship of the sizes and hypotenuse angles are further described below.

Figure 5 shows the backing member 140, which includes the cam elements 145, a proximal, relatively small end segment 144, a distal, relatively large end segment 146, and a throughbore 142. The cam elements 145 are attached to and/or otherwise extend from the proximal side of the proximal end segment 144 and are designed to mate with the cam features 135 shown in Figure 4A, e.g., when the covering body 120 is directed towards the backing member 140 during actuation, as explained elsewhere herein. Generally, the cam elements 145 and cam features 135 include ramped or angled surfaces 145a, 135a that interact to cause rotational motion of the shaft 130 relative to the backing member 140 in response to axial movement of the covering body 120 (or vice versa), as explained elsewhere herein. The proximal end segment 144 has a corresponding polygonal shape and size such that it matches the shape and size of the distal throughbore 118 of outer shell 110. Thus, the proximal end segment 144 may be received within the distal throughbore 118, thereby coupling rotation of the outer shell 110 to the backing member 140. The proximal end segment 144 (or other features of the backing member 140) may be substantially permanently attached to the outer shell 110, e.g., using an interference fit, one or more mating connectors (not shown), bonding with adhesive, sonic welding, and the like. It will also be appreciated that other interlocking features and/or shapes may be provided for coupling the outer shell 110 to the backing member 140.

The distal end segment 146 of the backing member 140 has an outer surface that has substantially the same polygonal shape and size of the outer surface 111 of the outer shell 110 in order for it to fit against the distal end 113 of the outer shell 110, e.g., to provide a continuous outer surface for the assembly 100. The throughbore 142 may be circular and/or have the same or substantially the same diameter and/or shape as the conduit 132 of the shaft 130.

Figures 6A-6H show the assembly 100 of the covering body 120, shaft 130, backing member 140, and elastic member 150. The outer shell 110 is shown in Figures 6A(1) and 6D(1), but has been excluded from Figures 6A(2) and 6D(2) for simplicity, e.g., to show the inner workings of the assembly 100. During manufacturing, the various components of the assembly 100 may be formed, e.g., by injection molding, machining, forming, and the like. The components may be formed from metal, plastic, and/or composite materials, as desired. Once the components are made, they may be assembled into the assembly 100.

In an exemplary method, the annular base 152 of the elastic member 150 may advanced over the fluid cap 136 onto the shaft 130, e.g., until the spokes 153 are disposed between the pegs 131. The shaft 130 may then be inserted into the covering body 120, e.g., until the hubs 151 of the elastic member are received in the recesses 121 in the outer mating surface 128. The deformable membrane 129 may be inserted into the male boss 127 of the covering body 120. The covering body 120 may then be inserted into the distal end 113 of the outer shell 110, e.g., after aligning the outer mating surface 128 with the distal throughbore 118 of the outer shell 110. The backing member 140 may then be connected to the outer shell 140 to close the distal throughbore 118. The backing member 140 may be substantially permanently attached to the outer shell 110, e.g., using cooperating connectors, bonding with adhesive, sonic welding, fusing, and the like.

Optionally, a section of tubing 170 may be attached to the shaft 130, e.g., through the backing member 140. Alternatively, a portion of the shaft 130 (not shown) may extend through the backing member 140, e.g., terminating in a connector (not shown), to allow a section of tubing or other device (also not shown) to be attached to the shaft 130. The resulting assembly 100 provides a covering body or inner housing 120 contained within the outer shell 110 and backing member or cover 140 such that the male boss 127 is disposed adjacent the Luer thread 114 in the outer shell 110. The covering body 120 may be movable axially, e.g., when the male boss 127 is contacted and pushed axially into the outer shell 110. The shaft 130 may be free to rotate within the assembly 100, e.g., limited by the interaction of the cam features 135 and cam elements 145 as described further below.

During use, as shown in Figures 6A-6H, a female Luer fitting 160 may be connected to the Luer thread 114 in the outer shell 110. The female Luer fitting 160 may be connected to a length of tubing, piece of equipment, container, and the like (not shown), which may be part of the IV or fluid line to which the assembly 100 is to be connected. The outer shell 110 (not shown) has the inner thread from male Luer 116 into which the female Luer fitting 160 may be threaded.

Initially, in the deactuated condition shown in Figures 6A(1) and 6A(2), the covering body 120 is biased away from the backing member 140. This is due to the securing of the hubs 151 of the elastic member 150 in the recesses 121 and the position of the pegs 131, as best seen in Figures 66B and 6G. Although the angled surfaces 135a, 145a of the cam features 135 and cam elements 145 may contact one another, the elastic member 150 may bias the shaft to slide up the surfaces 135a, 145a, i.e., away from the backing member 140 and towards the male Luer thread 114 on the outer shell 110. In this deactuated condition, the deactuated status indicators 125 may be aligned axially with the status windows 112.

As the female Luer fitting 160 threads into the outer shell 110, it contacts the male boss 127. Due to the tapered design of the male boss 127, the female Luer fitting 160 pushes the covering body 120 axially towards the distal end 113 of the outer shell 110, thereby directing the shaft 130 within the covering body 120 also to be directing axially towards the backing member 140. As the covering body 120 is pushed towards the backing member 140 by the female Luer fitting 160 contacting the male boss 127, the mating member 134 and shaft 130 are pushed along with the covering body 120. As the covering body 120 is directed axially, as best seen in Figure 6D(1), the actuated status indicators 126 become aligned with and can be seen through the status windows 112.

The resulting axial movement of the shaft 130 causes the cam features 135 to interact with the cam elements 145 to rotate the shaft 130 relative to the backing member 140 and, consequently, the outer shell 110 and covering body 120. More specifically, the ramped surfaces 135a of the cam features 135 slide along the ramped surfaces 145a of the cam elements 145, thereby translating the axial movement of the covering body 120 into axial and rotational movement of the shaft 130. The proximal tips of the cam elements 145 are lined up with the sloping corners of each hypotenuse of the cam features 135 before actuation, as shown in Figure 6A(2). As the shaft 130 is pushed distally, the cam features 135 are forced to mate with the cam elements 145, which forces the mating member 134 along with the rest of the shaft 130 to rotate.

As the shaft 130 rotates, the fluid cap 136 rotates inside the opening 129a in the deformable membrane 129. Before rotation, the polygonal shape of the fluid cap 136 is matched up with the polygonal shape of the opening 129a in the deformable membrane 129, thereby providing a substantially fluid-tight seal between the deformable member 129 and the fluid cap 136, i.e., sealing a fluid inside throughbore 133, as shown in Figure 6C. Rotation of the shaft 130 causes the fluid cap 136 and deformable membrane 129 to mismatch, thereby creating gaps 129b between the deformable membrane 129 and the fluid cap 136, as shown in Figure 6F. The elasticity of the deformable member 129 allows the fluid cap 136 to twist freely within the opening 129a. Once mismatched, gaps 129b are created between the fluid cap 136 and the deformable membrane 129, and fluid inside the throughbore 133 may now flow through the gaps 129b and into the female Luer fitting 160. At this point, the actuated status indicator 126 is seen through the status windows 112, as shown in Figure 6D(1).

The angle of each hypotenuse of the cam elements 145 and cam features 135 are such that when the shaft 130 is rotated to the point where the cam elements 145 are mated with the cam features 135, the fluid cap 136 is evenly mismatched with the deformable membrane 129. Evenly mismatched, in this sense, means that each of the points of the fluid cap 136, which is selectively shaped from the polygonal shapes mentioned above, are in substantially the midpoint of the sides of the opening 129a in the deformable membrane 129. Thus, with the use of pentagonally shaped members, there are five equal triangular gaps 129b formed when the assembly 100 is in the actuated condition. Each point of the pentagonally shaped fluid cap 136 is situated on the midpoint of the sides of the pentagonally shaped opening 129a in the deformable membrane 129, as best seen in Figure 6F. Although other degrees of deformation may work, the described deformation of the deformable membrane 129 may maximize the rate of fluid flow.

As the shaft 130 rotates, the spokes 153 of the elastic member 150 become stretched or deformed, as shown in Figures 6E and 6H, e.g., storing potential energy in the elastic member 150. The elastic member 150 is held in this stretched state by virtue of the mating between the cam features 145 and the cam elements 135. When the female Luer fitting 160 is disconnected, the energy stored in the spokes 153 of the elastic member 150 on the shaft 130 is not impeded anymore, and the shaft 130 is free to rotate back to its original position. During this deactuation process, the cam features 135 slide along the cam elements 145 causing the shaft 130 to move axially away from the backing member 140 as the shaft 130 rotates. This separation allows the shaft 130 and the covering body 120 to reassume their original relative rotational position, and causes the covering body 120 to move axially away from the backing member 140. After the covering body 120 is pushed back towards the proximal end 115 of the outer shell 110 through this deactuation process, the status windows 112 reveal the deactuated status indicator 125 once again.

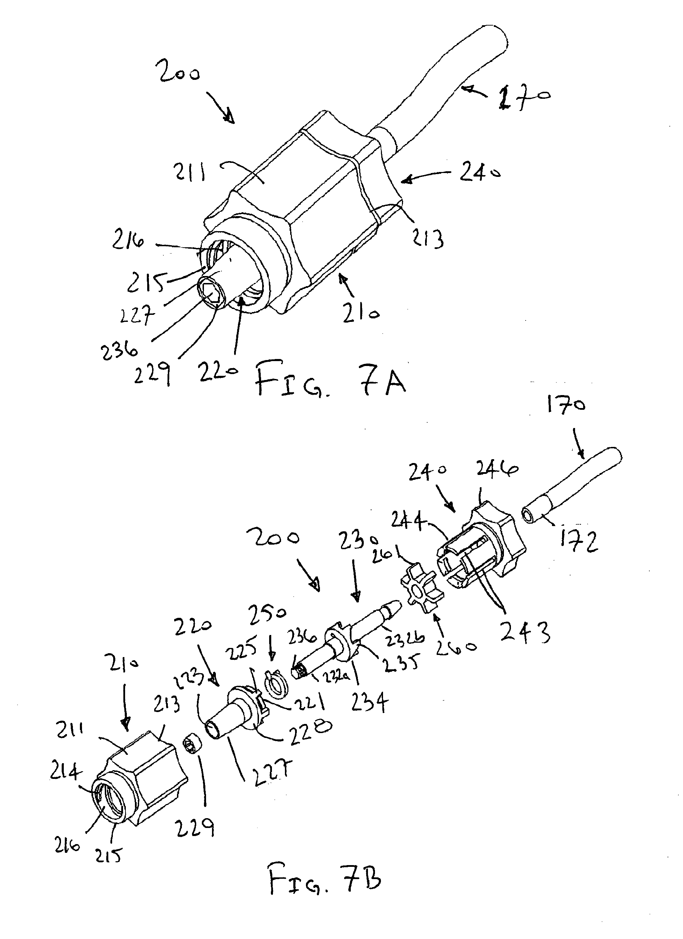

Turning to Figures 7A-12D, another embodiment of a valve/connector assembly 200 is shown that includes an outer shell 210 and backing member 240, together providing an outer package or housing for the assembly 200, a covering body or inner housing 220, a shaft, core pin, or tubular member 230, and an elastic member 250. These components are generally similar to the similarly identified components of the assembly 100 (with like components having their reference numbers increased by 100 for simplicity). Unlike the assembly 100, the assembly 200 also includes a gear-shaped camming element 260 that may be coupled to the outer shell 110 and backing member 240, as described further below.

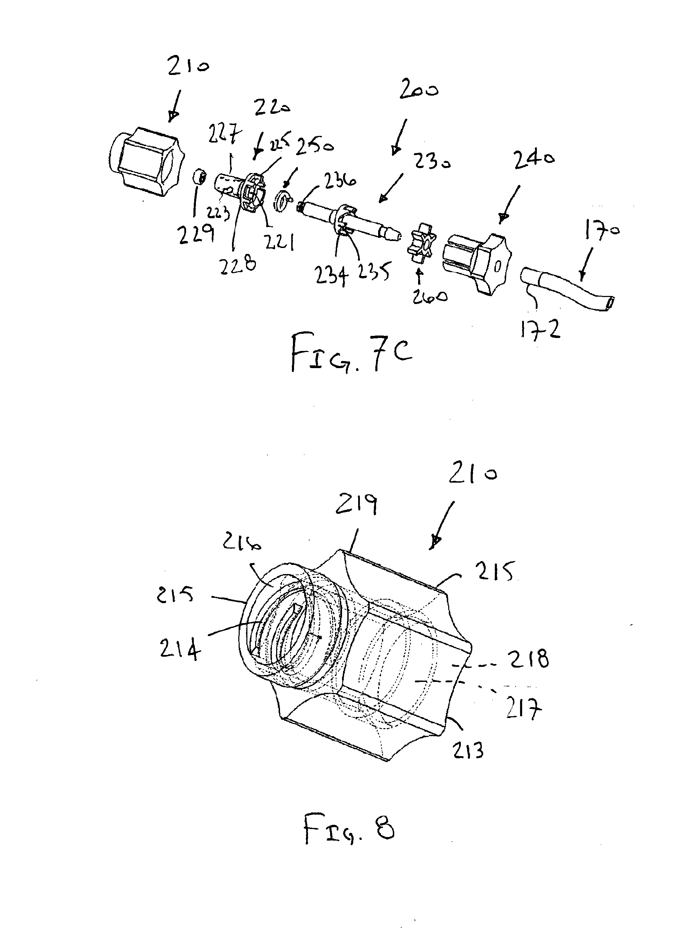

With particular reference to Figure 8, the outer shell 210 includes proximal and distal ends 215,213, an unthreaded portion 215 adjacent the distal end 213 with a distal throughbore 218 having an annular recess 217 therein, and a proximal throughbore 216 including a male Luer thread 214 therein adjacent the proximal end 215. As shown, the outer shell 210 has a hexagonal shaped outer surface 211, although, alternatively, the outer surface may have any other shape, similar to the previous embodiment. The outer surface 211 may facilitate a user gripping and/or manipulating the outer shell 210, e.g., to engage and/or disengage the male Luer thread 214 with a female Luer connector from a fluid line or other device (not shown).

The distal throughbore 218 extends from the distal end 213 of the outer shell 210 to intermediate location 219. The length, size, and/or shape of the distal throughbore 218 is sufficient to accommodate at least a portion of the covering body 220 and shaft 230 therein (not shown, see Figures 7A-7C). The diameter or other cross-section of the distal throughbore 218 may be larger than the proximal throughbore 216, e.g., to provide an abutment surface at the intermediate location 219 for limiting proximal movement of the covering body 220 into the distal throughbore 218, as explained further below.

The annular recess 217 may be disposed between the abutment surface at the intermediate location 219 and the distal end 213, e.g., closer to and/or immediately adjacent the distal end 213. The annular recess 217 may have a slightly larger diameter than the rest of the distal throughbore 218, e.g., having a height and diameter substantially similar to the camming element 260 so that the camming element 260 may be captured within the annular recess 217, e.g., to prevent removal of the camming element 260 while allowing it to spin within the annular recess 217. Thus, as described further below, the outer shell 310 may rotate relative to the camming element 260 and backing member 240, without separating the outer shell 210 from the camming element 260 and backing member 240 (but allowing the outer shell 210 to slide axially relative to the backing member 240).

The dimensions of the proximal throughbore 216 and male Luer thread 214 may be provided according to ISO standards for Luer connectors. Alternatively, other connectors (not shown) may be provided on the proximal end 215, if desired.

Returning to Figures 7B and 7C, the covering body 220 includes an elongated male boss 227 with a deformable membrane 229 attached thereto (similar to the previous embodiments), a throughbore 223, and a mating disc 228 including a plurality of teeth or tabs 225 and one or more lips or tabs 221. The male boss 227 may be dimensioned per ISO industry standards, e.g., having a cylindrical shape with a tapered proximal end. The male boss 227 and mating disc 228 of the covering body 220 may be integrally molded or otherwise formed from a single piece or may be separate pieces substantially permanently attached to one another.

Similar to the previous embodiments, the covering body 220 may be received within the outer shell 210, e.g., by inserting the male boss 227 through the distal throughbore 218 into the proximal throughbore 216, as shown in Figures 7A-7C. When the male boss 227 is received in the proximal throughbore 216, the mating disc 228 may be received in the distal throughbore 218, e.g., contacting the abutment surface at the intermediate location 219 (see Figure 8).

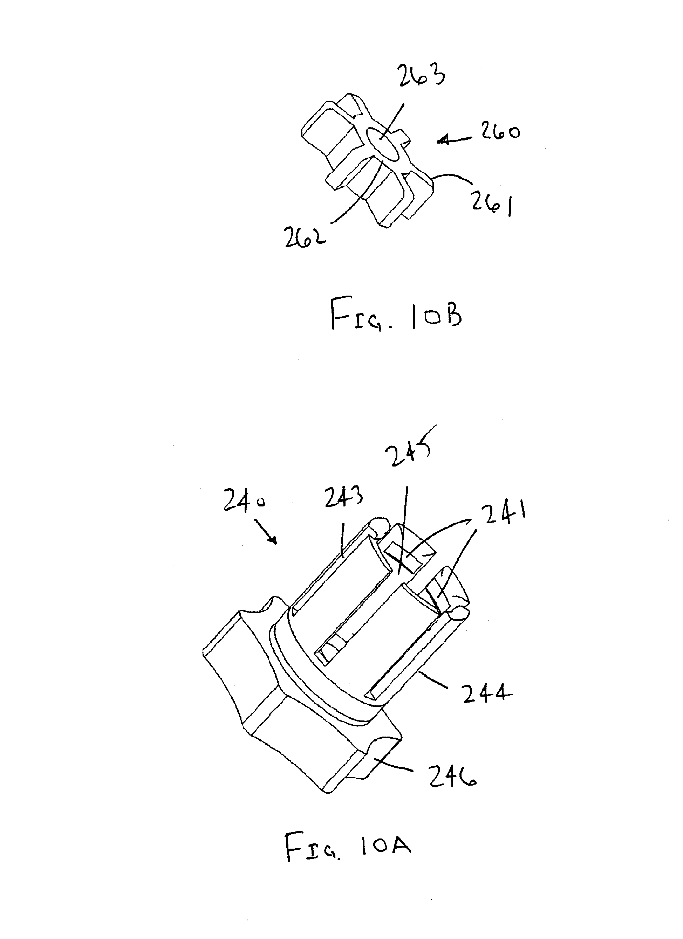

The mating disc 228 may have a diameter that substantially matches that of distal throughbore 218. The teeth 225 are attached to or otherwise extend from the distal side of the mating disc 228 and are configured to match gaps 243 in the backing member 240 (see Figure 10A), as described further below. Between the teeth 225 is a recessed portion of the mating disc 228 that includes the one or more tabs 221, which are configured to match and/or be received in indentation 241 of the backing member 240. As explained further below, the tabs 225 and tabs 221 may provide detents or connectors for attaching the covering body 220 to the backing member 240. It will be appreciated that other cooperating detents or connectors (not shown) may be provided on the covering body 220 and/or backing member 240 for this purpose.

The throughbore 223 runs the entire length of covering body 220, e.g., through the mating disc 228 and male boss 227. As shown, the throughbore 223 is substantially circular and may have a diameter similar to the outer diameter of conduit 232 of the shaft 230 (except for the proximal-most portion, which may have a polygonal shape for receiving the deformable membrane 229). The deformable membrane 229 may be attached to the male boss 237, e.g., inserted into the proximal portion of the throughbore 223 as shown in Figure 7A. The deformable membrane 229 may have a polygonal shaped hollow center that matches the size and shape of fluid cap 236 of the shaft 230, as described further elsewhere herein. The deformable membrane 229 may be fixedly attached to the inside of the male boss 227 such that the deformable membrane 229 cannot rotate therein, e.g., when the deformable membrane 229 is being deformed, similar to the previous embodiments.

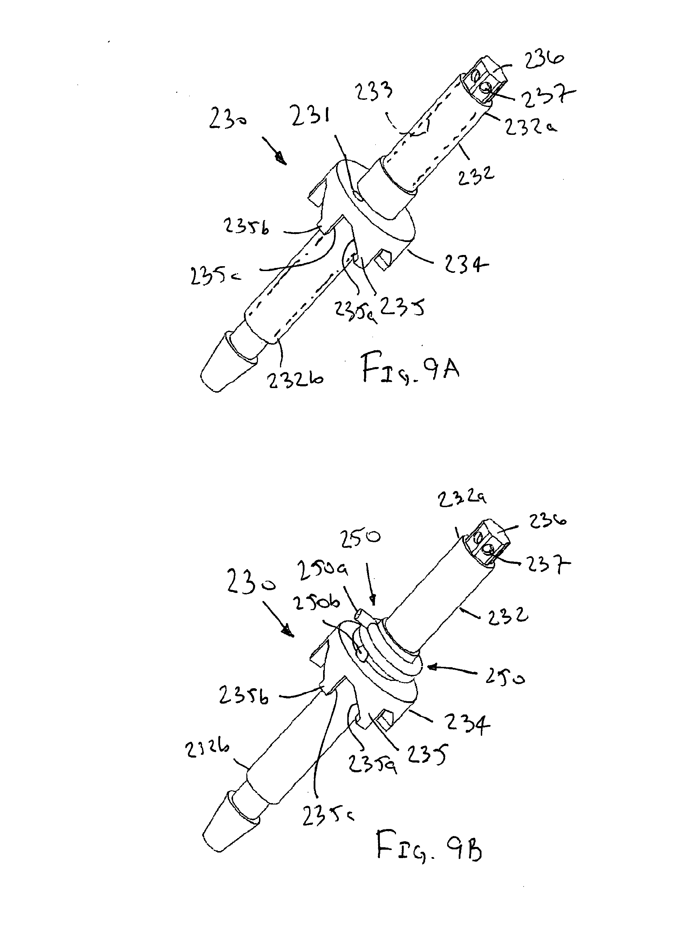

Turning to Figures 9A and 9B, the shaft 230 generally includes a conduit 232, a throughbore 233 extending between first and second ends 232a, 232b of the conduit 232, a mating member 234 with a plurality of cam features 235, and a fluid cap 236 with openings 237, similar to previous embodiments. The throughbore 233 extends through conduit 232, e.g., into the fluid cap 236. The fluid cap 236 has a polygonal shape that substantially matches the opening 229a in the deformable membrane 229, and may include one or more openings 237, e.g., on each face of its polygonal shape, e.g., to allow fluid to flow through the openings 237 after actuation of assembly 200, as described elsewhere herein.

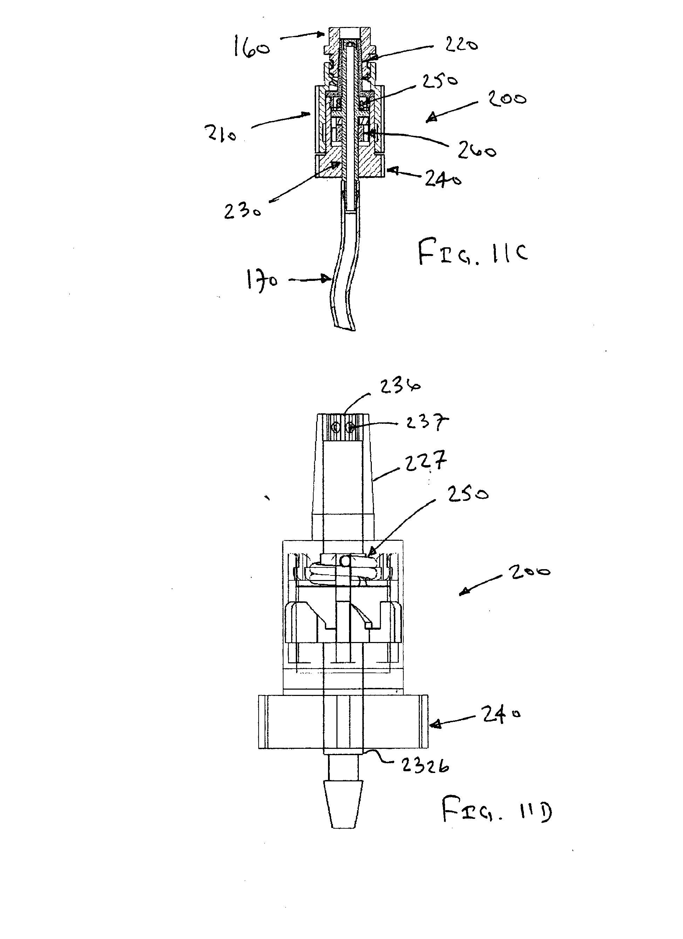

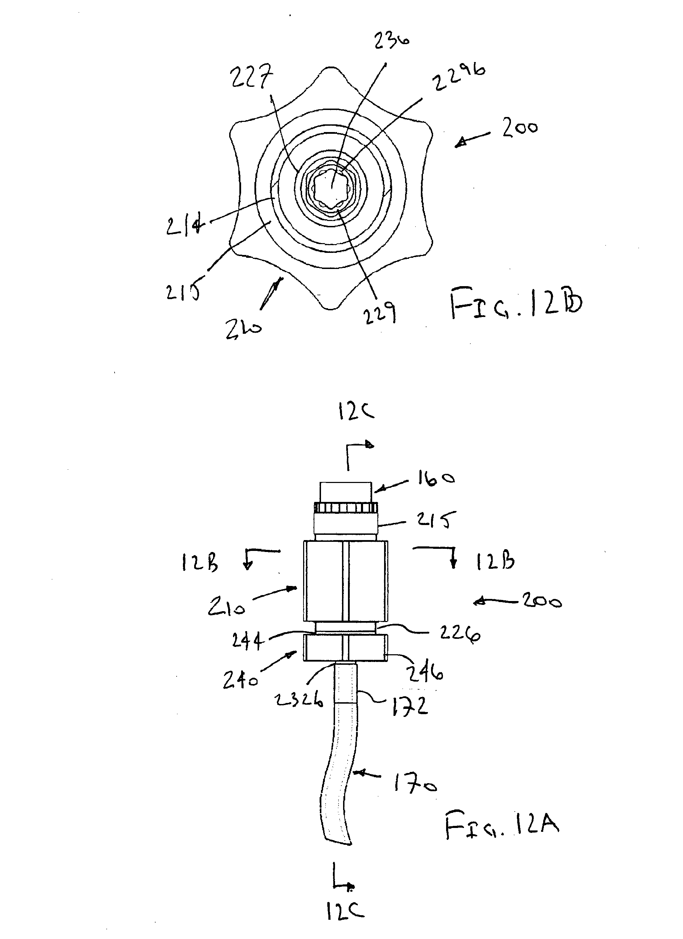

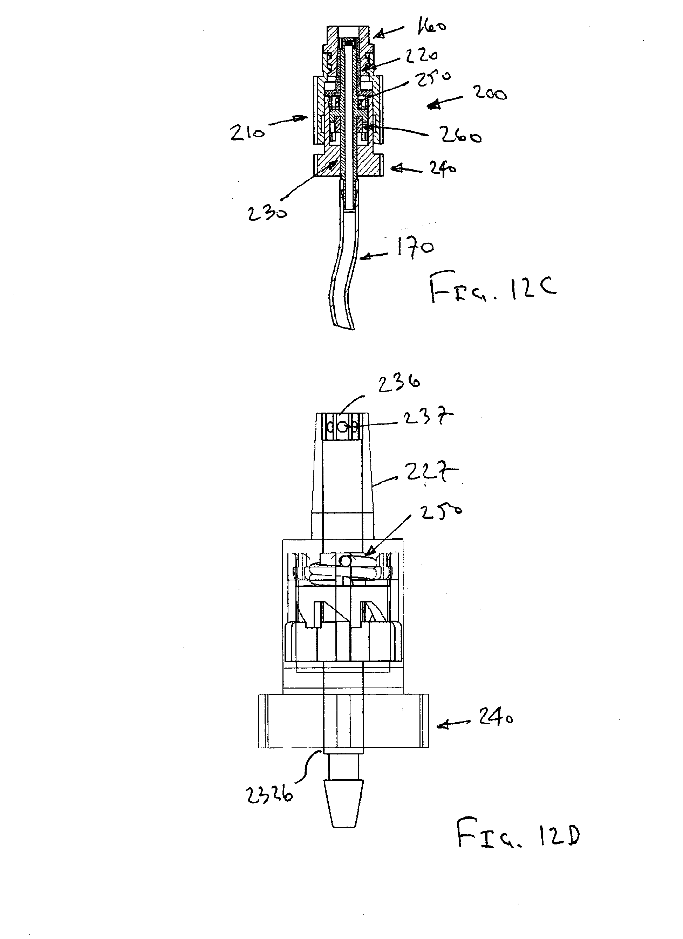

The mating member 234 may be located near the midpoint of the conduit 232. For example, the mating member 234 may be located along a length of the conduit 232 such that, when the shaft 230 is inserted into the covering body 220, as shown in Figures 11D and 12D, a distal side of the mating disc 228 may contact a proximal side of the mating member 234, e.g., to limit insertion of the shaft 230 into the covering body 220. The mating member 234 may include one or more holes or other anchor points 231, e.g., located on a proximal side of the mating member 234 to receive a portion of the elastic member 250 and/or otherwise limit movement of the elastic member 250 relative to the shaft 230.

The cam features 235 are located on the distal side of mating member 234, and generally include ramped surfaces 235a, similar to the previous embodiments. The cam features 235 may be configured to contact and/or otherwise interact with the camming element 260, e.g., to effect actuation and/or deactuation of the assembly 200, as described elsewhere herein. Similar to the assembly 100, the angle of each hypotenuse of the cam features 235 may be changed, e.g., to correspond to the selected polygonal shape for the fluid cap 236 and opening 229a, for example, to maximize fluid flow upon actuation. Unlike the assembly 100, each cam feature 235 may also include a tooth or extension 235b on the distal side, which is described further below.

Also shown in Figure 9B, the elastic member 250 is shown as a coil spring that includes first and second ends 250a, 250b. The elastic member 250 may be sized to be received around the conduit 232 of the shaft 230, e.g., above the mating member 234. As shown, the first end 250a may extend radially outwardly from the shaft 230, and the second end 250b is received in the hole 231 (best seen in Figure 9A). After assembly, the first end 250a of the elastic member 250 may be captured or otherwise engaged to the covering body 220 and/or backing member 240 to bias the shaft 230 relative to the covering body 220. Alternatively, other elastic members may be provided, similar to the other embodiments described herein.

Turning to Figure 10A, the backing member 240 includes a base portion 246, and an annular portion 244 extending from the base portion 246, together defining a throughbore 245 for receiving the shaft 232 and/or tubing 270 therein. The annular portion 244 includes a plurality of relatively narrow gaps or slots 243 extending axially from the base portion 246, thereby dividing the annular portion 244 into fingers. In addition, the annular portion 244 includes one or more annular recesses or grooves 241 disposed within the throughbore 245. For example, each finger may include a recess 241, which may be shaped to receive a corresponding tab 221 on the mating disc 228.

As shown, the base portion 246 includes an outer shape substantially similar to the outer shell 210, e.g., to define an outer housing for the assembly 200. In contrast, the annular portion 244 has a relatively smaller outer diameter or cross-section, e.g., similar to that of the mating disc 228. As best seen in Figures 11C and 12C, the annular portion 244 may be sized to be received in the distal throughbore 218 of the outer shell 210, as described further below.

The slots 234 are sized and/or shaped to received the teeth 225 on the covering body 220 and/or the extensions 235b on the camming features 235. For example, during assembly, the teeth 225 on the mating disc 228 may be received in respective slots 243 while the tabs 221 are received within the throughbore 245 of the annular portion 244, e.g., such that the tabs 221 are received in and/or otherwise engage the recesses 241, e.g., to substantially permanently attach the covering body 220 to the backing member 240. It will be appreciated that other mating connectors (not shown) may be provided on the covering body 220 (e.g., on the mating disc 228) and/or on the backing member 240 (e.g., on the annular portion 244) to substantially permanently or removably attach the covering body 220 to the backing member 240. In addition or alternatively, the covering body 220 may be attached to the backing member 240 by bonding with adhesive, sonic welding, fusing, and the like.

Turning to Figure 10B, the camming element 260 includes a plurality of spokes 261 extending from a central hub 262 including a passage 263 therethrough. The passage 263 has a diameter larger than the conduit 232 of the shaft 230, e.g., to allow the conduit 232 to be slidable through the camming element 260. The spacing and configuration of the spokes 261 may correspond to the slots 243 in the backing member 240, e.g., such that spokes 261 may be slidably received in the slots 243, thereby allowing the camming element 260 to slide axially relative to the backing member 240.

In addition, the spokes 261 may be sufficiently long such that, when the camming element 260 is inserted into the distal end 213 of the outer shell 210, the spokes 261 may be snapped, captured or otherwise received in the annular recess 217 such that camming element 260 is substantially fixed axially within the outer shell 210. The outer diameter defined by the spokes 261 may be slightly smaller than the annular recess 217, thereby allowing the camming element 260 and outer shell 210 to rotate relative to each other. The width of the spokes 261 may also substantially match or be slightly narrower than the height of the annular recess 217 in the outer shell 210.

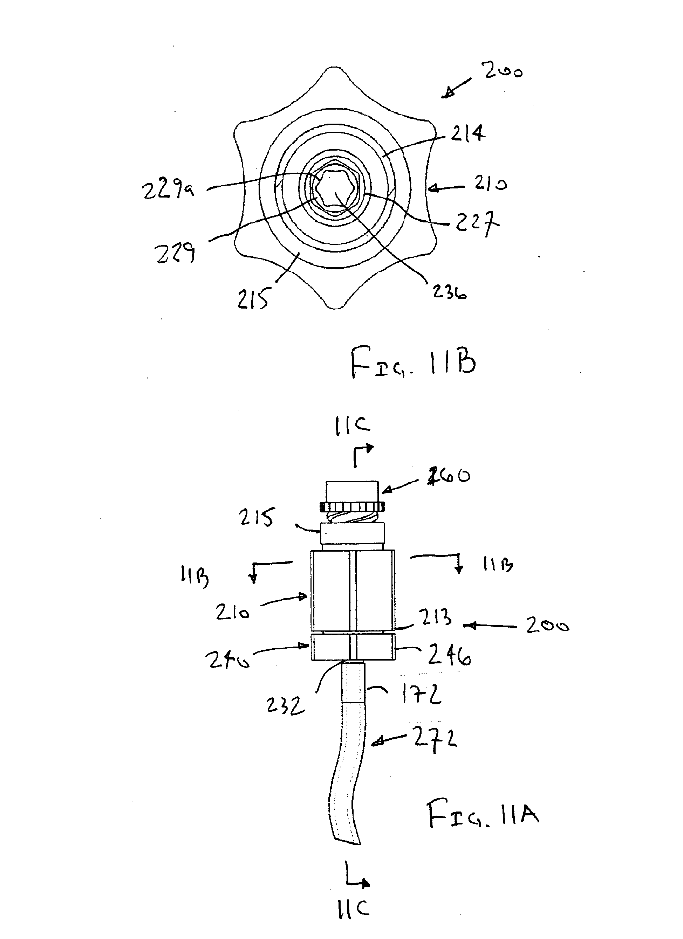

Figures 11A-12D show the assembly 200, including the outer shell 210, covering body 220, shaft 230, backing member 240, elastic member 250, and camming element 260 assembled together, in a deactuated or closed (no-flow) position (Figures 11A-11D) and an actuated or open (flow) position.

Before use, as shown in Figures 7A-7C, the components of the assembly 200 may be assembled together, e.g., during manufacturing. Although exemplary methods are described herein, it will be appreciated that the order of the various stages or steps and/or the particular steps used may be changed, as desired, e.g., based upon manufacturing convenience and/or other factors. With additional reference to Figure 9B, the elastic member 250 may be received around the shaft 230, e.g., around the conduit 232 above the mating member 234. The second end 250b of the elastic member 250 may be received in the hole 231, thereby preventing subsequent rotation of the elastic member 250 relative to the shaft 230.

The proximal end 232a of the shaft 230 may be inserted into the covering body 220, e.g., into the throughbore 223 through the mating disc 228 until the fluid cap 236 is received within the opening 229a of the deformable membrane 229, as best seen in Figure 7A. During this stage, the mating member 234 of the shaft 230 may be spaced apart from the mating disc 228 of the covering body 220, e.g., such that the elastic member 250 is spaced away from the mating disc 228. Alternatively, the relative length of the male boss 227 and conduit 232 may be such that the mating member 234 contacts or is received within the mating disc 228 (not shown). In this alternative, the covering body 220 may include an internal axial slot or peripheral hole (not shown) for receiving the first end 250a of the elastic member 250. This configuration may allow the shaft 230 to rotate within the covering body 220 yet the elastic member 250 may bias the shaft 230 to return to a desired rotational position (e.g., where the assembly 200 is closed, as explained elsewhere herein).

The covering body 220 (with the shaft 230 therein) may be inserted into the outer shell 210, e.g., into the distal throughbore 218 until the male boss 227 enters the proximal throughbore 216 and/or the mating disc 228 abuts the abutment surface at the intermediate location 219. The camming element 260 may be inserted over and around the distal end 232b of the shaft 230 and into the distal throughbore 218 of the outer shell 210, e.g., until the spokes 261 snap into or are otherwise captured or received within the annular recess 217.

The backing member 240 may be inserted into the distal end 213 of the outer shell 210, e.g., around the shaft 230 and camming element 260. This may involve aligning the slots 233 in the annular portion 244 of the backing member 240 with the spokes 261 on the camming element 260. The backing member 240 may be advanced until the annular portion 244 is connected to the mating disc 228 on the covering body 220. This connection may also require rotating the backing member 240 to ensure that the teeth 225 on the mating disc 228 are aligned with the slots 243 on the annular portion 244 and the tabs 221 enter the throughbore 245 of the annular portion 244, e.g., to engage the tabs 221 with the recesses 241.

If the assembly 200 is to be connected to tubing 170 (or another container or device, not shown), the end 172 of the tubing 170 may be connected to a connector on the distal end 232b of the shaft 230.

In an alternative procedure, the camming element 260 may be inserted into the throughbore 245 of the annular portion 244 of the backing member 240, i.e., after aligning the spokes 261 with the slots 243. The covering body 220, with the shaft 230 and elastic member 250 therein, may then be attached to the annular portion 244, e.g., by aligning the teeth 255, tabs 221, slots 243 and recesses 241, as described elsewhere herein. During this step, the first end 250a of the elastic member 250 may be received in one of the slots 243 in the annular portion 244. The outer shell 210 may then be received over this subassembly, e.g., by inserting the male boss 227 into the distal throughbore 218 of the outer shell 210 until the camming element 260 is captured in the annular recess 217.

During use, the assembly 200 may be provided as shown in Figures 7A and 11A-11D. In this condition, a fluid path through the assembly 200 may be biased to a closed position, e.g., with the shaft 230 in a rotational position such that the fluid cap 236 is aligned with and received in the opening 229a of the deformable membrane 229, as best seen in Figure 7A. The outer shell 210 may be freely rotated relative to the lacking member 240 and the internal components of the assembly 200. This may facilitate rotating the outer shell 210 to attach the assembly 200 to a fluid line or other device (not shown).

The outer shell 210 may also be free to move axially, although the axial movement is limited by the movement of the camming element 260 axially within the annular portion 244 of the backing member 240. For example, the outer shell 210 may be directed distally until the camming member 260 and/or outer shell 210 contacts the base portion 246. In this distal position, the male boss 227 may extend to and/or partially out of the proximal end 215 of the outer shell 210. This position may facilitate cleaning the male boss 227, fluid cap 236, and/or deformable membrane 229, e.g., during use. In addition, the outer shell 210 may be directed proximally away from the base portion 246 of the backing member 240 until the camming element 260 contacts the cam features 235 on the mating member 234 of the shaft 230.

Turning to Figure 11A, a female Luer 160 is shown, which includes an external Luer thread that is partially inserted into the proximal end 215 of the outer shell 210. In this position, the outer shell 210 may remain movable axially some distance relative to the backing member 240.

Turning to Figures 12A-12D, as a female Luer 160 is fully threaded into the outer shell 210, it pushes the male boss 227 toward the distal end 213 of the outer shell 210. This causes the covering body 220 and shaft 230 to move toward the distal end 213 of the outer shell 210. Stated differently, as the female Luer 160 is threaded into the proximal end 215 of the outer shell 210, the outer shell 210 may be pulled proximally away from the male boss 227 and the base portion 246 of the backing member 240, thereby moving the camming element 260 away from the base portion 246 and towards the mating member 234 on the shaft 230.

This causes the camming element 260 to contact the cam features 235 on the shaft 230, i.e., the cam features 235 are pushed against the spokes 261 of the camming element 260, which causes the shaft 230 to rotate. The angle of each hypotenuse and size of cam features 235 may be such that, when the shaft 230 is rotated to the point where the spokes 261 bottom out and abut vertical walls 235c adjacent the cam features 235. In this position, the fluid cap 236 is mismatched with the deformable membrane 229, i.e., the fluid cap 236 has rotated relative to the opening 229a to create gaps 229b between the fluid cap 236 and the deformable membrane 229. The gaps 229b open a fluid path between the throughbore 233 in the shaft 230 and the proximal throughbore 216 of the outer shell 210, i.e., into the Luer fitting 160 attached to the assembly 200.

For example, the fluid cap 236 may be substantially evenly mismatched with the deformable membrane 229, i.e., such that each of the points of the fluid cap 236 are in substantially the midpoint of the sides of the opening 229a in the deformable membrane 229. As shown in Figures 11B and 12B, for example, using hexagonally shaped members, there are six equal substantially triangular gaps 229b formed between the fluid cap 236 and the deformable membrane 229 when the assembly 200 is in the actuated state, i.e., because each point of the hexagonally shaped fluid cap 236 is situated on the midpoint of the sides of the hexagonally shaped opening 229a in the deformable membrane 229. Although other degrees of deformation may work, the described deformation of the deformable membrane may maximize the rate of fluid flow.

In addition, when the female Luer connector 160 is tightened into the proximal end 215 of the outer shell 210, a region 226 of the annular portion 244 of the backing member 240 may become exposed. If desired, an actuated status indicator 226 may be provided on the annular portion 244, as shown in Figure 12A, which may become exposed only when the female Luer connector 160 is finally tightened to the outer shell 210. The status indicator 226 may include a color (such as green), one or more words, and the like, similar to other embodiments herein, which may provide the user visual confirmation that the assembly 200 has achieved the actuated position in which the flow path is open. Optionally, the region between the actuated status indicator 226 and the base portion 240 may include another status indicator (not shown), such as a contrasting color (e.g., red), which may provide a visual indication to the user that the assembly 200 has not yet been actuated.

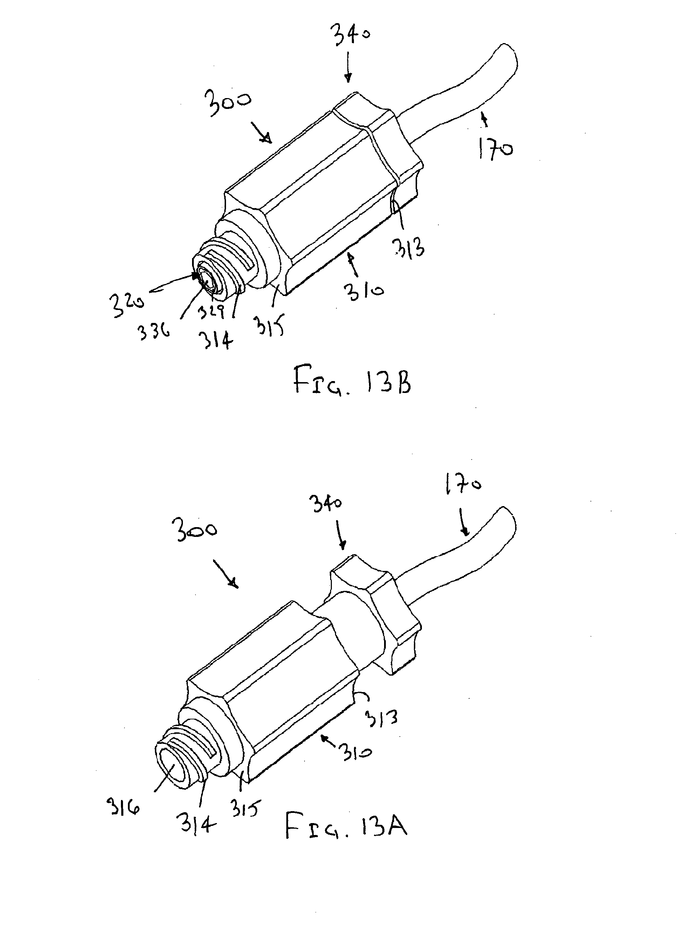

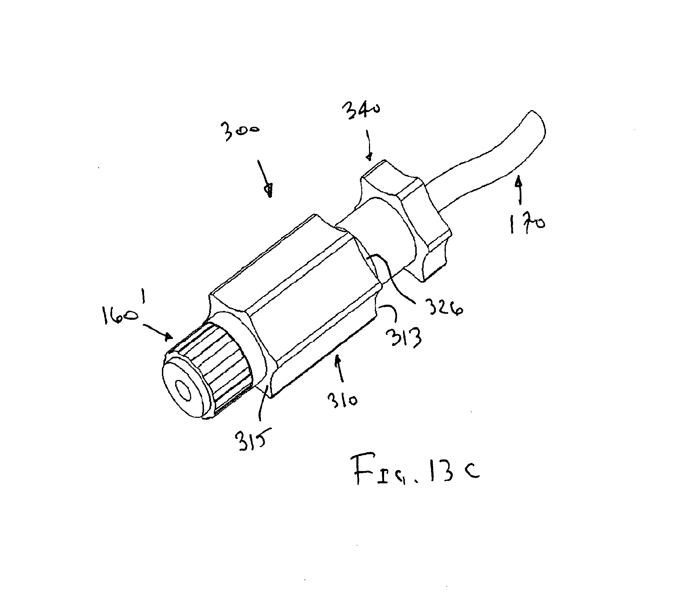

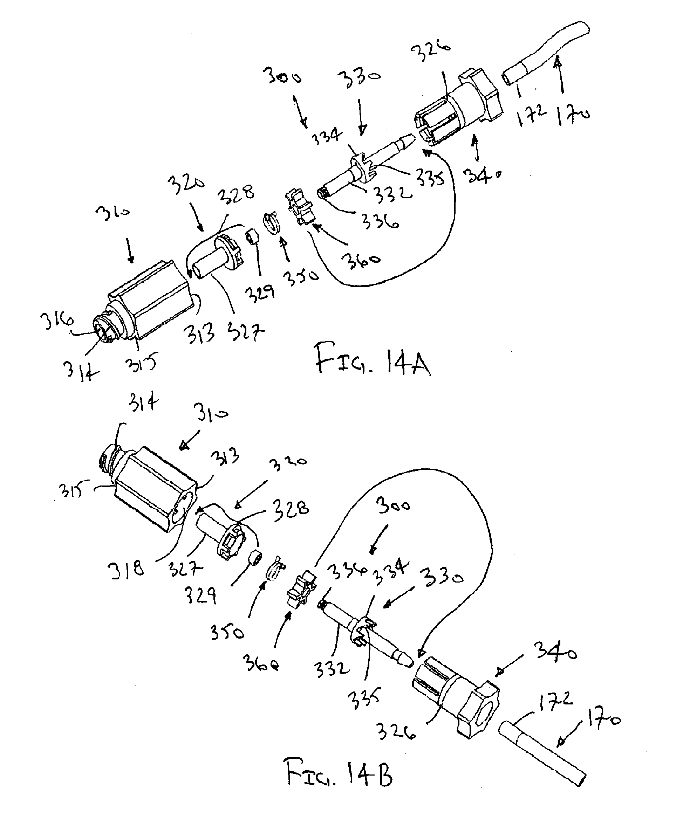

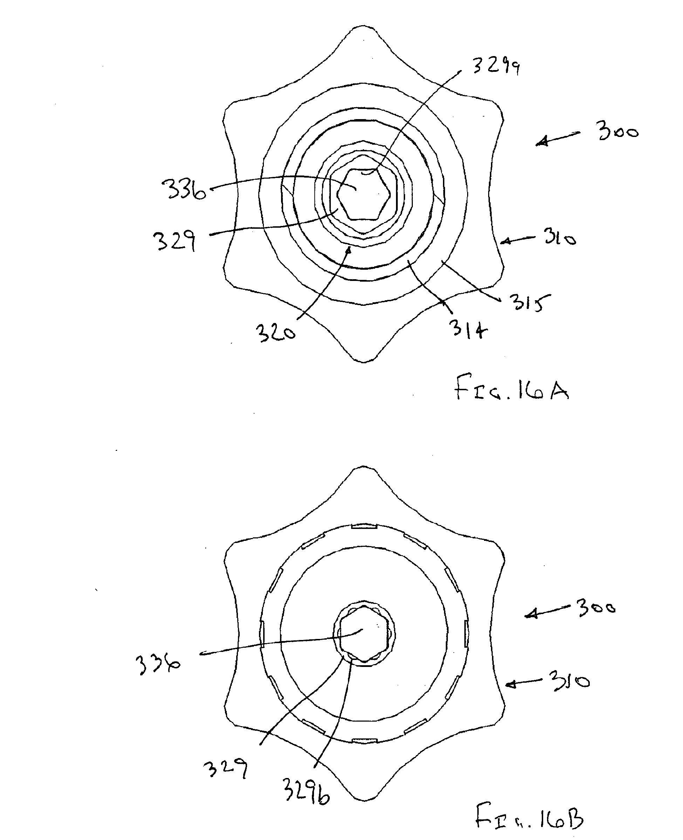

Turning to Figures 13A-16B, yet another exemplary embodiment of a valve/connector assembly 300 is shown that includes the same general components as the assembly 200. The corresponding structures of the assembly 300 function in the same manner as described above for the assemblies 100, 200. As best seen in Figures 14A and 14B, the assembly 300 includes an outer shell 310 with a female Luer thread 314, a covering body or inner housing 320 with a deformable membrane 329, a shaft or core pin 330 with an elastic member, i.e., spring 350, and a backing member 340, generally similar to the apparatus 200. The outer shell 310 is structurally distinct from the outer shell 210 in assembly 200, and includes a proximal throughbore 316, an unthreaded portion 315, a tapered portion 317, a distal throughbore 318, and a female Luer thread 314.

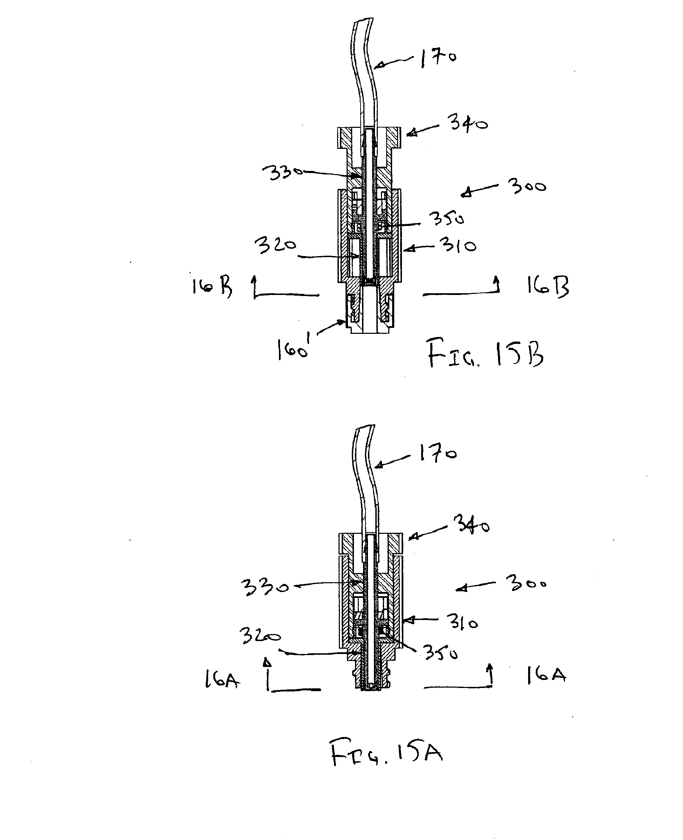

Generally, the components, assembly, use, and operation of the assembly 300 is similar to the previous embodiments. Unlike the previous embodiments, however, the outer shell 300 includes a female Luer connector and thread 314 for mating with a male Luer connector 160' (shown in Figures 15A and 15B), e.g., communicating with a fluid line or other device (not shown).

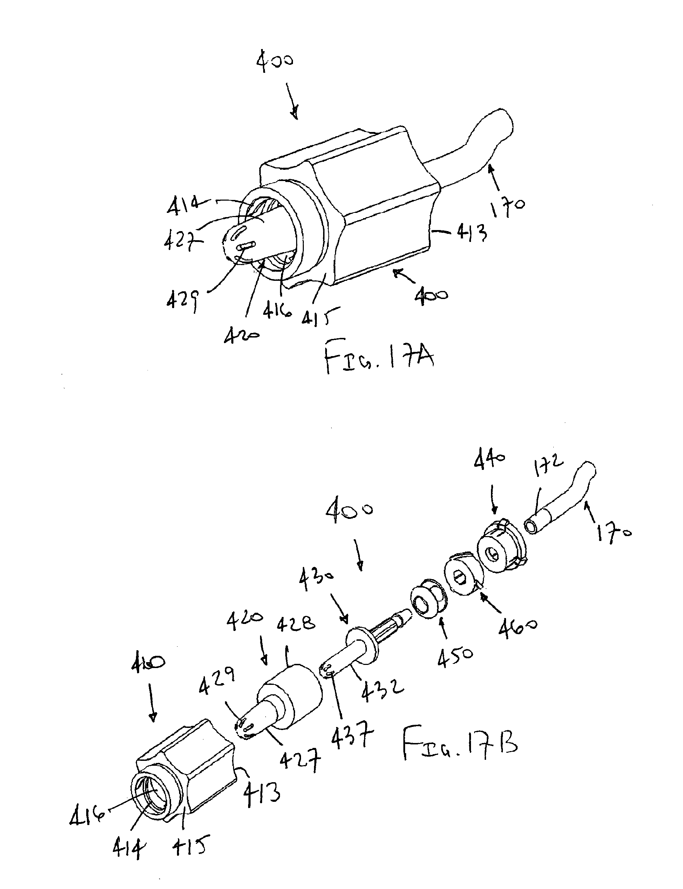

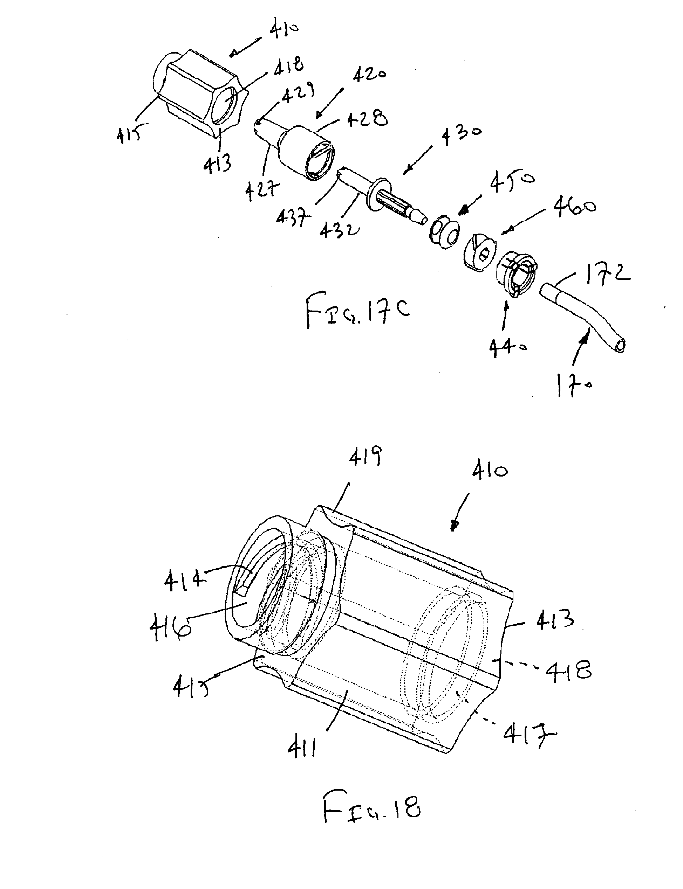

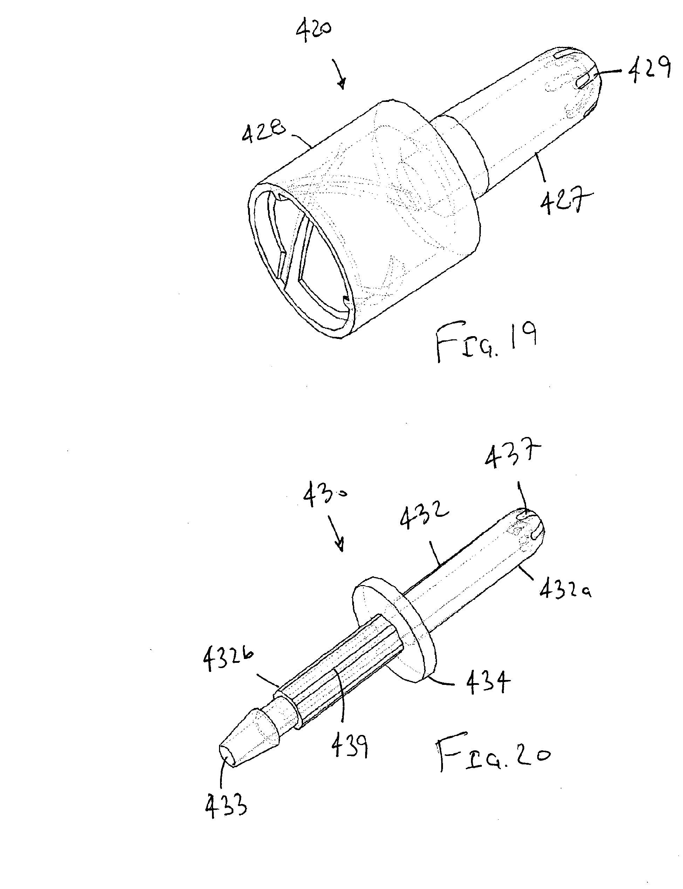

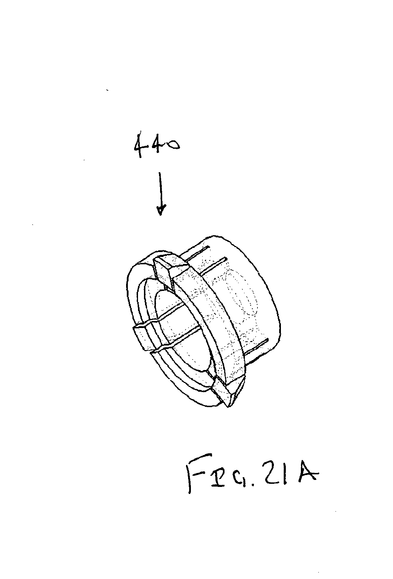

Turning to Figures 17A-23C, still another exemplary embodiment of a valve/connector assembly 400 is shown that includes an outer shell 410, a covering body or inner housing 420, a shaft, core pin, or tubular member 430, a backing member 440, an elastic member 450, and a camming element 460. Generally, the components, assembly, use, and operation of the assembly 400 are similar to the previous embodiments.

Unlike the previous embodiments, the covering body 420 includes a closed cap including a plurality of openings 429 in the side thereof. In addition, the shaft 430 includes a closed proximal end 432a including a plurality of openings 437 in a side wall thereof. The elastic member 450 includes a compression member, such as one or more spring washers, which may bias the covering body 420 into the proximal end 415 of the outer shell 410. It will be appreciated that these versions of the covering body 420/shaft 430 or elastic member 450 may be utilized in other embodiments disclosed herein or may be replaced with similar components from the other embodiments herein.

In addition, the shaft 430 includes a distal end 432b that has a noncircular cross-section, e.g., a polygonal shape, similar to a passage through the elastic member 460. The camming element 460 includes one or more helical ridges that are shaped similar to helical grooves in the mating member 428 of the covering body 420. It will be appreciated that other mating helical features may be provided on or in the camming element 460 and covering body 420, e.g., to provide a camming mechanism that translates relative axial movement between the covering body 420 and the shaft 430 into rotational motion.

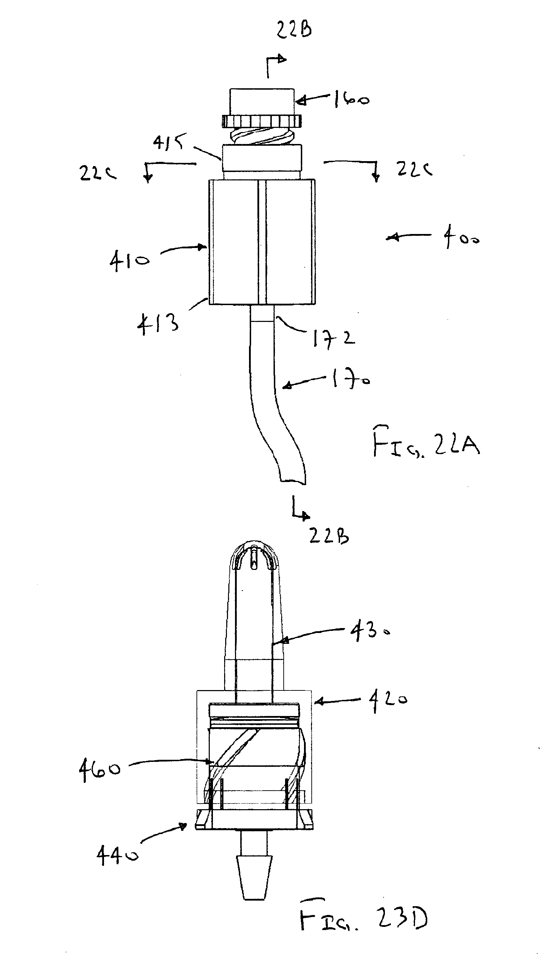

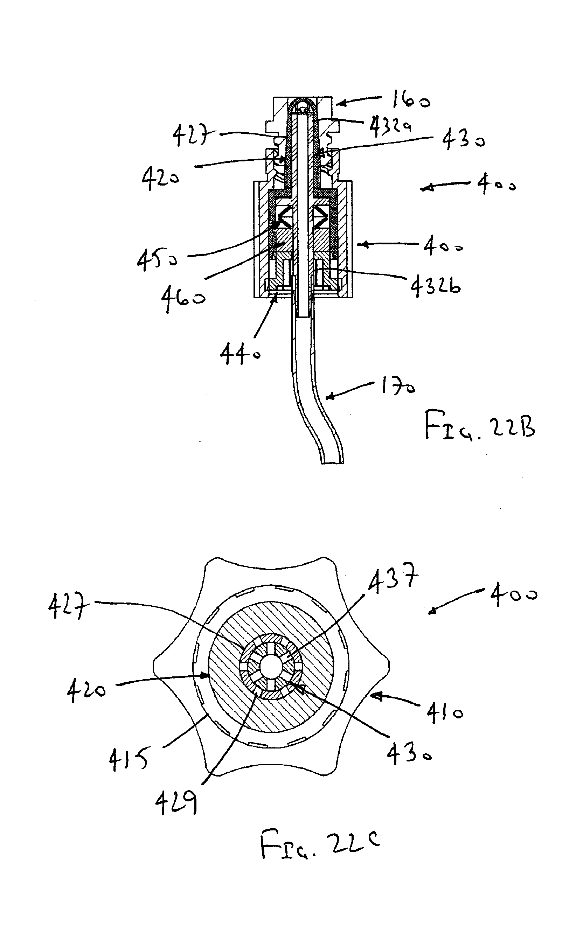

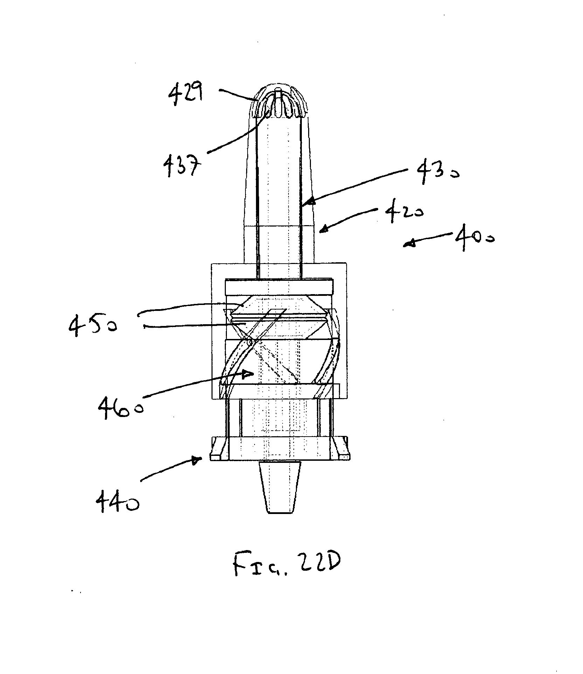

Turning to Figures 22A-22D, the assembly 400 is shown in the closed position in which the openings 437, 429 in the shaft 430 and covering body 420 are out of alignment with one another, thereby closing a fluid path between the throughbore in the shaft 430 and the proximal end 415 of the outer shell 410.

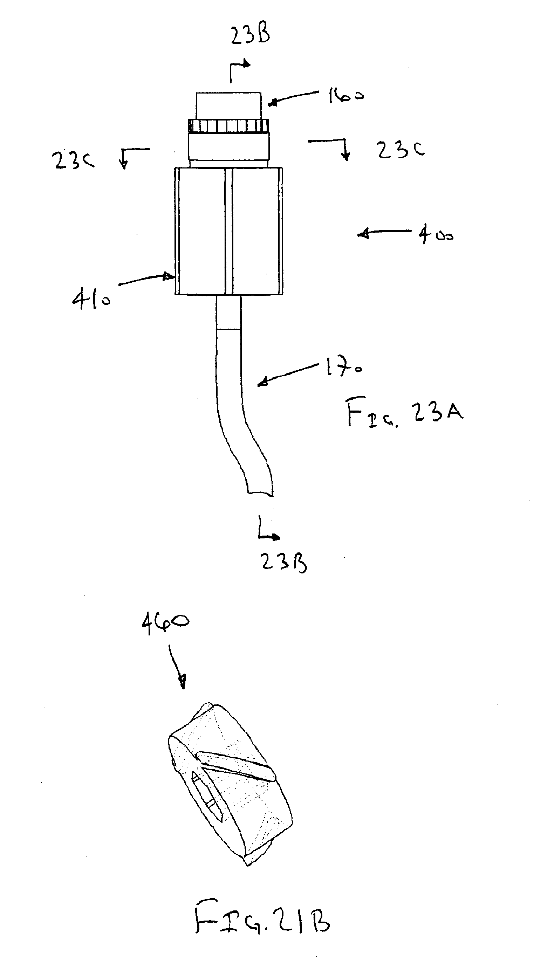

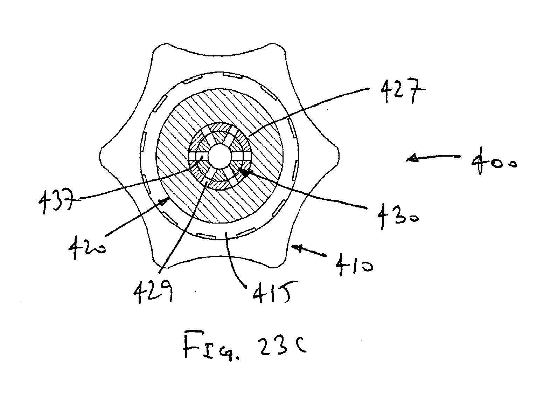

Turning to Figures 23A-23D, when a connector, e.g., a female Luer fitting 160, is connected to the proximal end 415 of the outer shell 410, the covering body 420 is directed distally towards the backing member 440. This causes the camming element 460 and, consequently, the shaft 430 to rotate relative to the covering body 420 due to the interaction of the helical camming features, thereby aligning the openings 437, 429 and opening the fluid path through the assembly 400.

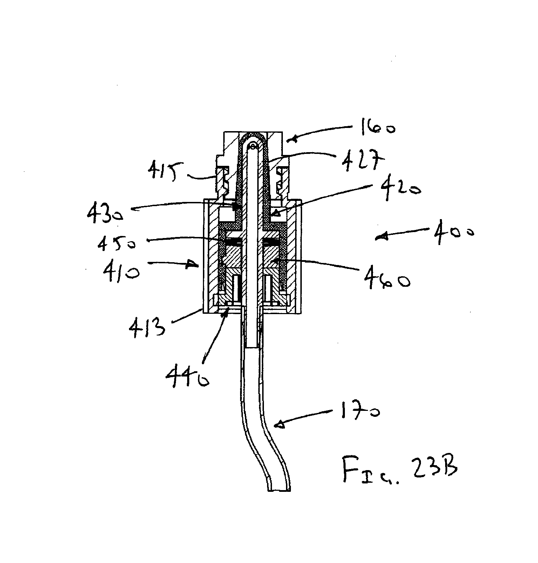

When the covering body 420 is directed distally, the elastic member 450 may be compressed, as shown in Figure 23B. When the connector 160 is disconnected from the assembly 410, the elastic member 450 may resiliently expand, thereby biasing the covering body 420 to move proximally away from the backing member 440, and causing the shaft 430 to rotate to automatically close the fluid path through the assembly 400.

The foregoing disclosure of the exemplary embodiments has been presented for purposes of illustration and description. It is not intended to be exhaustive or to limit the invention to the precise forms disclosed. Many variations and modifications of the embodiments described herein will be apparent to one of ordinary skill in the art in light of the above disclosure. For example, elimination of some components, such as the flexible sleeve that deforms in actuation, is possible and within the scope of the present invention. Another method may include allowing the core to rotate and deform the tip of the male Luer without the need for a sleeve. The scope of the invention is to be defined only by the claims appended hereto, and by their equivalents.

Further, in describing representative embodiments, the specification may have presented the method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims.

While the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents and alternatives falling within the scope of the appended claims.

## Claims

1. A connector assembly (100, 200, 300, 400) for controlling flow in a fluid line, comprising:
an outer shell (110, 140, 210, 240, 310, 410, 400, 440) comprising a first end (115, 215, 415), a second end (113, 213, 413), and a passage (116, 118, 216, 218, 316, 318, 416, 418) extending therebetween, the first end comprising a connector (114, 214, 314, 414) for connecting the assembly to a fluid line;
and **characterized by**;
an inner housing (120, 220, 320, 420) disposed within the outer shell and comprising a boss (127, 227, 327, 427) disposed adjacent the first end, the inner housing being movable axially within the outer shell between a first position adjacent the first end and a second position further from the first end than the first position when a device is connected to the connector on the first end of the outer shell;
a tubular member (130, 230, 330, 430) disposed within the inner housing and comprising a fluid passage (133, 233) therein extending between the second end of the outer shell and the boss of the inner housing, the tubular member carried by the inner housing such that the tubular member moves axially as the inner housing moves between the first and second positions; and
cam features (135, 145, 235, 260, 460) on one of the inner housing and the outer shell and on the tubular member for causing the tubular member to rotate as the inner housing moves between the first and second positions, thereby opening a fluid path between the fluid passage in the tubular member and the first end of the outer shell to allow fluid flow through the assembly with the device connected to the first end of the outer shell.

2. The connector assembly of claim 1, wherein the cam features (135, 145, 235, 260) are on the outer shell and the tubular member.

3. The connector assembly of claim 1 or 2, wherein the outer shell comprises a bezel (110, 210) and a backing member (140, 240), and wherein the cam features comprises one or more cam elements (145, 260) on the backing member that interact with one or more mating cam features (135, 235) on the tubular member.

4. The connector assembly of any one of claims 1-3, wherein the inner housing comprises a deformable membrane (129, 229, 329) substantially closing the boss, the tubular member comprising a fluid cap (136, 236) coupled to the deformable membrane to open one or more gaps (129b, 229b) between the deformable membrane and the fluid cap when the tubular member is rotated between the first and second positions.

5. The connector assembly of any one of claims 1-3, wherein the inner housing comprises one or more openings (429), and wherein the tubular member (430) comprises one or more openings (437), wherein the one or more openings in the inner housing are offset from the one or more openings in the tubular member in the first position, and wherein the one or more openings in the inner housing are aligned from the one or more openings in the tubular member in the second position, thereby allowing fluid flow through the one or more openings.

6. The connector assembly of claim 1, wherein the cam features are on the inner housing and the tubular member.

7. The connector assembly of any preceding claim, wherein the inner housing is biased to the first position, thereby biasing the tubular member to close the fluid path.

8. The connector assembly of claim 7, wherein the inner housing is biased to the first position by an elastic member (150, 250, 350, 450).

9. The connector assembly of claim 7, wherein the outer shell comprises a backing member, and wherein the inner housing is attached to the backing member, and wherein the outer shell is movable axially and rotationally relative to the backing member.

10. The connector assembly of claim 9, wherein the tubular member comprises a camming member coupled to a shaft, the camming member comprising the cam features cooperating with cam features on the inner housing such that axial movement of the inner housing member relative to the outer shell causes the camming member to rotate relative to the inner housing, thereby causing the shaft to rotate relative to the inner housing.

11. The connector assembly of claim 4, wherein the fluid cap has a geometrical shape substantially similar to a hollow center of the deformable membrane, and has at least one opening on the sides of the fluid cap contacting the deformable membrane;
wherein initially the openings are occluded by the deformable membrane; and
whereby rotation of the fluid cap inside the deformable membrane creates a plurality of gaps between the fluid cap and the hollow center to allow fluid flow.

12. The connector assembly of any preceding claim, wherein the boss is of the Luer type.

13. The connecting assembly of any preceding claims, wherein the outer shell has a polygon shape and status windows (112) revealing when the assembly is in an actuated or deactuated state.

14. The connecting assembly of claim 9, further comprising a status indicator (226, 326) on the backing member to provide a visual confirmation that the fluid path is open.

15. The connector assembly of claim 8, wherein the elastic member is an elastic band, an elastic disk, a torsional energy story element, a bellevile type spring, or a gear-shaped member.

## Patentansprüche

1. Verbinderbaugruppe (100, 200, 300, 400) zur Steuerung des Flusses in einer Fluidleitung, die aufweist:
eine äußeres Gehäuse (110, 140, 210, 240, 310, 410, 400, 440), das ein erstes Ende (115, 215, 415), ein zweites Ende (113, 213, 413) und eine sich dazwischen erstreckende Passage (116, 118, 216, 218, 316, 318, 416, 418) aufweist, wobei das erste Ende einen Verbinder (114, 214, 314, 414) umfasst, um die Baugruppe mit einer Fluidleitung zu verbinden;
und **gekennzeichnet ist durch**:
ein inneres Gehäuse (120, 220, 320, 420), das innerhalb des äußeren Gehäuses angeordnet ist und eine an das erste Ende angrenzend angeordnete Nabe (127, 227, 327, 427) aufweist, wobei das innere Gehäuse innerhalb des äußeren Gehäuses zwischen einer dem ersten Ende benachbarten Position und einer zweiten Position axial beweglich ist, die von dem ersten Ende weiter entfernt ist als die erste Position, wenn mit dem Verbinder an dem ersten Ende des äußeren Gehäuses eine Vorrichtung verbunden ist;
ein rohrförmiges Element (130, 230, 330, 430), das innerhalb des inneren Gehäuses angeordnet ist und darin eine Fluidpassage (133, 233) aufweist, die sich zwischen dem zweiten Ende des äußern Gehäuses und der Nabe des inneren Gehäuses erstreckt, wobei das rohrförmige Element so von dem inneren Gehäuse getragen wird, dass sich das rohrförmige Element bei einer Bewegung des inneren Gehäuses zwischen der ersten und der zweiten Position axial bewegt; und
Kurvenführungen (135, 145, 235, 260, 460) an dem inneren Gehäuse oder dem äußeren Gehäuse und dem rohrförmigen Element, um ein Drehen des rohrförmigen Elements bei einer Bewegung des inneren Gehäuses zwischen der ersten und zweiten Position zu bewirken, wodurch sich zwischen der Fluidpassage in dem rohrförmigen Element und dem ersten Ende des äußeren Gehäuses ein Fluidpfad öffnet, der einen Fluidfluss durch die Baugruppe ermöglicht, wenn die Vorrichtung mit dem ersten Ende des äußeren Gehäuses verbunden ist.

2. Verbinderbaugruppe nach Anspruch 1, wobei sich die Kurvenführungen (135, 145, 235, 260, 460) an dem äußeren Gehäuse und an dem rohrförmigen Element befinden.

3. Verbinderbaugruppe nach Anspruch 1 oder 2, wobei das äußere Gehäuse einen Reif (110, 210) und ein Abschlusselement (140, 240) aufweist und wobei die Kurvenführungen an dem Abschlusselement ein oder mehrere Kurvenelemente (145, 260) aufweisen, die mit einem oder mehreren passenden Kurvenführungen (135, 235) an dem rohrförmigen Element zusammenwirken.

4. Verbinderbaugruppe nach einem der Ansprüche 1 bis 3, wobei das innere Gehäuse eine verformbare Dichtung (129, 229, 329) umfasst, die die Nabe im Wesentlichen verschließt, das rohrförmige Element eine Fluidkappe (136, 236) aufweist, die so mit der verformbaren Dichtung verbunden ist, dass sich zwischen der verformbaren Dichtung und der Fluidkappe bei einem Drehen des rohrförmigen Elements zwischen der ersten und zweiten Position eine oder mehrere Lücken (129b, 229b) öffnen.

5. Verbinderbaugruppe nach einem der Ansprüche 1 bis 3, wobei das innere Gehäuse eine oder mehrere Öffnungen (429) aufweist und wobei das rohrförmige Element (430) eine oder mehrere Öffnungen (437) aufweist, wobei in der ersten Position die eine oder die mehreren Öffnungen in dem inneren Gehäuse zu der einen oder den mehreren Öffnungen in dem rohrförmigen Element versetzt sind, und wobei sich in der zweiten Position die eine oder die mehreren Öffnungen in dem inneren Gehäuse und die eine oder die mehreren Öffnungen in dem rohrförmigen Element decken, wodurch ein Fluidfluss durch die eine oder die mehreren Öffnungen möglich ist.

6. Verbinderbaugruppe nach Anspruch 1, wobei sich die Kurvenführungen am inneren Gehäuse und am rohrförmigen Element befinden.

7. Verbinderbaugruppe nach einem der vorhergehenden Ansprüche, wobei das innere Gehäuse in die erste Position vorgespannt ist, wodurch das rohrförmige Element zum Schließen des Fluidpfads vorgespannt ist.

8. Verbinderbaugruppe nach Anspruch 7, wobei das innere Gehäuse durch ein elastisches Element (150, 250, 350, 450) in die erste Position vorgespannt ist.

9. Verbinderbaugruppe nach Anspruch 7, wobei das äußere Gehäuse ein Abschlusselement aufweist, wobei das innere Gehäuse an dem Abschlusselement befestigt ist und wobei das äußere Gehäuse relativ zu dem Abschlusselement axial und drehend bewegbar ist.

10. Verbinderbaugruppe nach Anspruch 9, wobei das rohrförmige Element ein Kurvenführungselement aufweist, das mit einem Schaft verbunden ist, wobei das Kurvenführungselement die Kurvenführungen aufweist, die mit den Kurvenführungen an dem inneren Gehäuse so zusammenwirken, dass eine axiale Bewegung des inneren Gehäuseelements relativ zu dem äußeren Gehäuse ein Drehen des Kurvenführungselements relativ zum inneren Gehäuse bewirkt, sodass der Schaft relativ zu dem inneren Gehäuse rotiert.

11. Verbinderbaugruppe nach Anspruch 4, wobei die geometrische Form der Fluidkappe dem zentralen Hohlraum der verformbaren Dichtung im Wesentlichen gleicht und wobei die Fluidkappe zumindest eine Öffnung an ihren Seiten aufweist, die die verformbare Dichtung berühren;
wobei die Öffnungen anfangs durch die verformbare Dichtung verschlossen sind; und
wobei durch ein Drehen der Fluidkappe innerhalb der verformbaren Dichtung mehrere Lücken zwischen der Fluidkappe und dem zentralen Hohlraum geschaffen werden, um einen Fluidfluss zu ermöglichen.

12. Verbinderbaugruppe nach einem der vorhergehenden Ansprüche, wobei die Nabe vom Luer-Typ ist.

13. Verbinderbaugruppe nach einem der vorhergehenden Ansprüche, wobei das äußere Gehäuse polygonförmig ist und ein Statusfenster (112) aufweist, das anzeigt, ob sich die Baugruppe in einem betätigten oder in einem nicht betätigten Zustand befindet.

14. Verbinderbaugruppe nach Anspruch 9, die ferner an dem Abschlusselement eine Statusanzeige (226, 326) aufweist, um eine visuelle Bestätigung darüber zu erhalten, dass der Fluidpfad geöffnet ist.

15. Verbinderbaugruppe nach Anspruch 8, wobei das elastische Element ein elastisches Band, eine elastische Scheibe, ein energiespeicherndes Torsionselement, eine Feder vom Typ einer Tellerfeder oder ein flügelradförmiges Element ist.

## Revendications

1. Ensemble de raccord (100, 200, 300, 400) pour commander l'écoulement dans une conduite de fluide, comprenant :
une coque externe (110, 140, 210, 240, 310, 410, 400, 440) comprenant une première extrémité (115, 215, 415), une deuxième extrémité (113, 213, 413), et un passage (116, 118, 216, 218, 316, 318, 416, 418) s'étendant entre celles-ci, la première extrémité comprenant un raccord (114, 214, 314, 414) pour relier l'ensemble à une conduite de fluide ;
et **caractérisé par** :
un logement interne (120, 220, 320, 420) disposé à l'intérieur de la coque externe et comprenant un bossage (127, 227, 327, 427) disposé de manière adjacente à la première extrémité, le logement interne pouvant se déplacer axialement à l'intérieur de la coque externe entre une première position adjacente à la première extrémité et une deuxième position plus éloignée de la première extrémité que la première position lorsqu'un dispositif est relié au raccord sur la première extrémité de la coque externe ;
un élément tubulaire (130, 230, 330, 430) disposé à l'intérieur du logement interne et comprenant un passage de fluide (133, 233) dedans s'étendant entre la deuxième extrémité de la coque externe et le bossage du logement interne, l'élément tubulaire étant supporté par le logement interne de sorte que l'élément tubulaire se déplace axialement à mesure que le logement interne se déplace entre les première et deuxième positions ; et
des éléments de came (135, 145, 235, 260, 460) sur l'un(e) du logement interne et de la coque externe et sur l'élément tubulaire pour amener l'élément tubulaire à tourner à mesure que le logement interne se déplace entre les première et deuxième positions, ouvrant ainsi un trajet de fluide entre le passage de fluide dans l'élément tubulaire et la première extrémité de la coque externe pour permettre l'écoulement de fluide à travers l'ensemble avec le dispositif relié à la première extrémité de la coque externe.

2. Ensemble de raccord de la revendication 1, dans lequel les éléments de came (135, 145, 235, 260) sont sur la coque externe et l'élément tubulaire.

3. Ensemble de raccord de la revendication 1 ou 2, dans lequel la coque externe comprend une collerette (110, 210) et un élément de support (140, 240), et où les éléments de came comprennent un ou plusieurs organe(s) de came (145, 260) sur l'élément de support qui interagit/interagissent avec un ou plusieurs élément(s) de came d'accouplement (135, 235) sur l'élément tubulaire.

4. Ensemble de raccord de l'une quelconque des revendications 1 à 3, dans lequel le logement interne comprend une membrane déformable (129, 229, 329) fermant essentiellement le bossage, l'élément tubulaire comprenant un bouchon de fluide (136, 236) couplé à la membrane déformable pour ouvrir un ou plusieurs espace(s) (129b, 229b) entre la membrane déformable et le bouchon de fluide lorsque l'élément tubulaire est mis en rotation entre les première et deuxième positions.

5. Ensemble de raccord de l'une quelconque des revendications 1 à 3, dans lequel le logement interne comprend une ou plusieurs ouverture(s) (429), et où l'élément tubulaire (430) comprend une ou plusieurs ouverture(s) (437), où la ou les plusieurs ouverture(s) dans le logement interne est/sont décalée(s) par rapport à la ou aux plusieurs ouverture(s) dans l'élément tubulaire dans la première position, et où la ou les plusieurs ouverture(s) dans le logement interne est/sont alignée(s) par rapport à la ou aux plusieurs ouverture(s) dans l'élément tubulaire dans la deuxième position, permettant ainsi l'écoulement de fluide à travers la ou les plusieurs ouverture(s).

6. Ensemble de raccord de la revendication 1, dans lequel les éléments de came sont sur le logement interne et l'élément tubulaire.

7. Ensemble de raccord de l'une des revendications précédentes, dans lequel le logement interne est sollicité vers la première position, sollicitant ainsi l'élément tubulaire pour fermer le trajet de fluide.

8. Ensemble de raccord de la revendication 7, dans lequel le logement interne est sollicité vers la première position par un élément élastique (150, 250, 350, 450).

9. Ensemble de raccord de la revendication 7, dans lequel la coque externe comprend un élément de support, et où le logement interne est fixé à l'élément de support, et où la coque externe peut se déplacer axialement et en rotation par rapport à l'élément de support.

10. Ensemble de raccord de la revendication 9, dans lequel l'élément tubulaire comprend un élément à effet de came couplé à un arbre, l'élément à effet de came comprenant les éléments de came coopérant avec des éléments de came sur le logement interne de sorte que le mouvement axial de l'élément de logement interne par rapport à la coque externe amène l'élément à effet de came à tourner par rapport au logement interne, amenant ainsi l'arbre à tourner par rapport au logement interne.

11. Ensemble de raccord de la revendication 4, dans lequel le bouchon de fluide a une forme géométrique essentiellement similaire à un centre creux de la membrane déformable, et a au moins une ouverture sur les côtés du bouchon de fluide venant en contact avec la membrane déformable ;
dans lequel initialement les ouvertures sont obstruées par la membrane déformable ; et
moyennant quoi la rotation du bouchon de fluide à l'intérieur de la membrane déformable crée une pluralité d'espaces entre le bouchon de fluide et le centre creux pour permettre l'écoulement de fluide.

12. Ensemble de raccord de l'une des revendications précédentes, dans lequel le bossage est du type Luer.

13. Ensemble de raccordement de l'une des revendications précédentes, dans lequel la coque externe a une forme polygonale et des fenêtres d'état (112) révélant quand l'ensemble est dans un état actionné ou non actionné.

14. Ensemble de raccordement de la revendication 9, comprenant en outre un indicateur d'état (226, 326) sur l'élément de support pour fournir une confirmation visuelle que le trajet de fluide est ouvert.

15. Ensemble de raccord de la revendication 8, dans lequel l'élément élastique est une bande élastique, un disque élastique, un élément de stockage d'énergie de torsion, un ressort de type Belleville, ou un élément en forme d'engrenage.
